# EUROPEAN PATENT SPECIFICATION

(11) **EP 3 738 620 B1**
(45) Date of publication and mention of the grant of the patent: **08.03.2023**
(21) Application number: 18899625.0
(22) Date of filing: 28.12.2018
(51) Int. Cl.: A61L 27/16, A61F 2/16, A61L 27/44, A61L 27/50, C08F 220/12

(54) **INTRAOCULAR LENS**
INTRAOKULARE LINSE
LENTILLE INTRAOCULAIRE

(30) Priority: 09.01.2018 JP 2018001522
(43) Date of publication of application: 18.11.2020
(73) Proprietor: Nidek Co., Ltd., Gamagori-shi, Aichi, 443-0038 (JP)
(72) Inventor: TADA, Takahiro, Gamagori-shi, Aichi 443-0038 (JP); YOKOTA, Satoshi, Gamagori-shi, Aichi 443-0038 (JP); MURASE, Kyoko, Gamagori-shi, Aichi 443-0038 (JP); NAGASAKA, Shinji, Gamagori-shi, Aichi 443-0038 (JP); SUNADA, Tsutomu, Gamagori-shi, Aichi 443-0038 (JP)
(74) Representative: Kuhnen & Wacker Patent- und Rechtsanwaltsbüro PartG mbB
(86) International application number: PCT/JP2018/048609
(87) International publication number: WO 2019/138952

(56) References cited:
- JP-A- H07 157 523
- JP-A- H08 173 522
- JP-A- H08 173 522
- JP-A- H08 308 921
- JP-A- H08 308 921
- JP-A- 2002 017 845
- JP-A- 2002 017 845
- US-A- 5 519 070

## Description

### Technical Field

The present invention relates to an intraocular lens used by being inserted into an eye. The present application claims priority to Japanese Patent Application No. 2018-001522 filed on January 9, 2018.

### Background Art

One conventional method widely resorted to for the treatment of cataracts involves excising a white, cloudy crystalline lens from the lens capsule, and inserting into the capsule an intraocular lens (IOL) having refractive power, in place of the crystalline lens. Such an intraocular lens is provided with an optical portion that exhibits refractive power, and a support portion that fixes the position of the optical portion within the capsule. In order to keep an incision wound on the eyeball as small as possible at the time of insertion of the intraocular lens, for instance a single-piece intraocular lens in which for example an optical portion and a support portion are integrally molded with each other is prepared and is inserted into the eye, in a folded state, using an insertion instrument called an injector. The inserted intraocular lens reverts again to its original shape, inside the capsule, with the position of the intraocular lens within the capsule being stabilized through pressing of the support portion against the capsule (see for instance Patent Literature 1 to 5). Moreover, Patent Literatures 6 to 9 disclose ocular lenses according to prior art. Patent literature 8 describes a flexible intraocular lens obtained by copolymerizing one or more acrylate based monomer selected from a list including 2-hydroxy-3-phenoxypropyl acrylate or 2-methoxyethyl acrylate , an aromatic ring-containing methacrylate (phenoxyethyl methacrylate) and 1,3-butadiene glycol dimethacrylate. An UV absorber can be added to the composition. The ophthalmic lens material exhibits low tackiness.

### Citation List

### Patent Literature

Patent Literature 1: JP 2001-151913 A
Patent Literature 2: JP 2001-269358 A
Patent Literature 3: JP 2001-340444 A
Patent Literature 4: JP 2002-325829 A
Patent Literature 5: JP 2011-136154 A
Patent Literature 6: JP 2002-017845 A
Patent Literature 7: JP H08-308921 A
Patent Literature 8: JP H08-173522 A
Patent Literature 9: US 5 519 070 A

### Summary of Invention

### Technical Problem

The intraocular lens is thus required to exhibit, besides eye refractive power, also foldable flexibility as well as an elastic restoring force (shape restoring force) back to an original shape. However, hydrophobic intraocular lens materials capable of exhibiting these properties also have tackiness on account of their flexibility. Therefore, problems arose in that the intraocular lens folded in the eye did not readily spread smoothly, and was prone to adhering to a surgical instrument such as a forceps. Patent Literature 1 to 5 propose subjecting the intraocular lens to a surface treatment such as a roughening treatment or plasma irradiation, in order to reduce the tackiness of the intraocular lens. However, all these methods are disadvantageous in terms of the increased time and labor required accompanying the greater number of processes involved.

Therefore, it is an object of the present invention to provide an intraocular lens having reduced surface tackiness.

### Solution to Problem

The intraocular lens provided by the art disclosed herein is composed of a (meth)acrylate-based copolymer that includes an acrylate-based monomer component A and a methacrylate-based monomer component B, as constituent components. The intraocular lens includes a surface portion that includes a surface of the copolymer, and a central portion closer to a center than the surface portion. The surface portion forms a methacrylate-rich layer containing a higher content of the monomer component B, as a monomer component, than that in the central portion.

In such a configuration, the surface portion of the intraocular lens is constituted by a methacrylate-rich layer. In other words, the proportion of the acrylate-based monomer component A included in the surface portion is reduced, and the tackiness that an acrylic-based polymer itself may inherently exhibit is relatively suppressed herein at the surface portion as compared with other portions. As a result an intraocular lens is provided having reduced surface tackiness and that is superior in handleability and operability, and which can easily unfold within the eye and revert to its original shape, for instance even when folded at the time of insertion into the eye.

In the present description, the term "(meth)acrylate" refers comprehensively to both acrylate and methacrylate, for instance, "(meth)acrylic acid" refers comprehensively to both acrylic acid and methacrylic acid. Further, the "(meth)acrylate-based polymer" denotes a polymer that has, in the polymer structure, monomer units derived from an acrylate-based monomer and monomer units derived from a methacrylate-based monomer. Typically, the term "(meth)acrylate-based polymer" refers herein to a polymer in which the proportion of the total of monomer units derived from the acrylate-based monomer and the methacrylate-based monomer exceeds 50 mass%.

In a preferred embodiment of the intraocular lens disclosed herein, the alkoxy group-containing acrylate is included, the methacrylate-based monomer component B includes an aromatic ring-containing methacrylate; and the intraocular lens further includes a hydrophilic hydroxy group-containing acrylate as a monomer component.

An intraocular lens is provided which, in addition to the above effect, combines flexibility shape recovery with a reduction in glistenings, while having a configuration that includes a hydroxy group-containing acrylate.

In a preferred embodiment of the intraocular lens disclosed herein, the alkoxy group-containing acrylate includes an acrylate represented by General formula (1) below, and the aromatic ring-containing methacrylate includes a methacrylate represented by General formula (2) below. In a Raman spectroscopic analysis spectrum at an excitation wavelength of 532 nm, when a ratio of an intensity I_{Arm} of a Raman peak derived from an aromatic ring observed in the vicinity of 1003 cm⁻¹ with respect to an intensity I_{CH} of a Raman peak derived from a C-H bond observed in the vicinity of 1450 cm⁻¹ is represented by R = I_{Arm}/I_{CH}, the ratio Rs₁ at the surface portion of the intraocular lens and the ratio Rc₁ at the central portion of the intraocular lens satisfy the relationship of Rs₁ ≥ 1.1 × Rc₁. In Formula (1), R1 is a methyl group or ethyl group, and n is an integer from 1 to 4. In Formula (2), R2 is a C₁₋₈ linear or branched alkylene group, and X is absent or represents an oxygen atom.

By virtue of the above configuration, an intraocular lens is provided in which surface tackiness is reduced more reliably, and which combines flexibility and shape recovery with a reduction in glistenings.

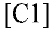

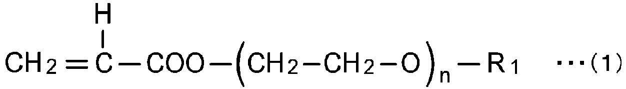

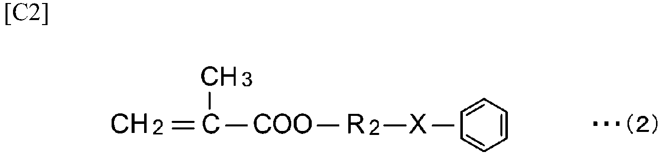

In a preferred embodiment of the intraocular lens disclosed herein, among the monomer components that constitute the copolymer, the aromatic ring-containing methacrylate is contained in an amount of at least 40 mass% and at most 52 mass%, the alkoxy group-containing acrylate is contained in an amount of at least 35 mass% and at most 46 mass%, and the hydroxy group-containing acrylate is contained in an amount of at least 8 mass% and at most 12 mass%.

By virtue of this configuration an intraocular lens is provided that combines yet better flexibility and shape recovery with a reduction in glistenings.

In a preferred embodiment of the intraocular lens disclosed herein, the acrylate-based monomer component A includes a compound represented by General formula (3) below, the methacrylate-based monomer component B includes a compound represented by General formula (4) below. In a Raman spectroscopic analysis spectrum at an excitation wavelength of 532 nm, when a ratio of an intensity I_{Arm} of a Raman peak derived from an aromatic ring observed in the vicinity of 1000 cm⁻¹ with respect to an intensity I_{CH} of a Raman peak derived from a C-H bond observed in the vicinity of 1452 cm⁻¹ is represented by R = I_{Arm}/I_{CH}, the ratio Rs₂ at the surface portion of the intraocular lens and the ratio Rc₂ at the central portion of the intraocular lens satisfy the relationship of Rs₂ ≤ 0.95 × Rc₂. In Formula (3), R3 is a C₁₋₆ linear or branched alkylene group, and Y is absent or represents an oxygen atom.

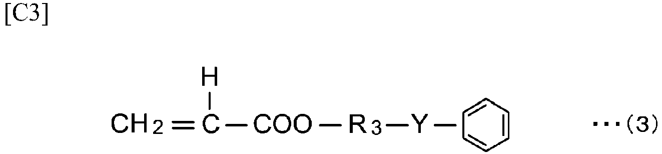

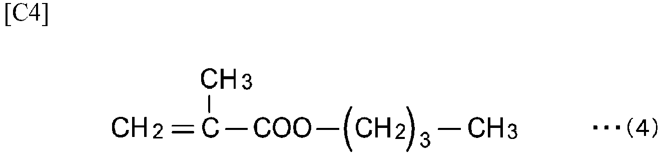

In the above configuration, the refractive index of the copolymer can be increased by virtue of the fact that the acrylate-based monomer component A includes a compound having an aromatic ring. The proportion of the monomer component A in the surface portion is kept significantly lower than that in the central portion. This is preferable since, as a result, the intraocular lens can be made thinner than conventional ones, while maintaining refractive power. Moreover, flexibility, a shape restoring force and low tackiness can be ensured even with a thinner intraocular lens. As a result an intraocular lens is provided that boasts good handleability and poses less of a burden on the practitioner.

In a preferred embodiment of the intraocular lens disclosed herein, among the monomer components that constitute the copolymer, the proportion of the acrylate-based monomer component A in all monomer components is at least 42 mass% and at most 62 mass%, and the proportion of the methacrylate-based monomer component B in all monomer components is at least 35 mass% and at most 55 mass%. That is, although the intraocular lens disclosed herein contains overall a large amount of the acrylate-based monomer component A, the proportion of the monomer component A is however kept low in the surface portion. As a result the tackiness of the surface portion can be conveniently suppressed while flexibility and a shape restoring force can be suitably imparted to the intraocular lens.

In a preferred embodiment of the intraocular lens disclosed herein, the surface portion has a refractive index of at least 1.505 and less than 1.51, and the central portion has a refractive index of at least 1.51 and at most 1.53. For instance prescribing thus the intraocular lens to have a high refractive index is preferable since in that case a predetermined refractive index can be achieved with a smaller thickness.

In a preferred embodiment of the intraocular lens disclosed herein, the ratio R changes continuously from the central portion towards the surface of the intraocular lens. In other words, the intraocular lens does not have a separately formed methacrylate-rich layer, of higher proportion of the methacrylate-based monomer component B, that is layered on the surface of the central portion; instead, the intraocular lens has a graded structure in which the proportion of the monomer component A and the monomer component B changes gradually. The central portion and the surface portion are formed integrally with each other. As a result there is provided a surface portion that forms the methacrylate-rich layer, without adversely affecting the field of view.

In a preferred embodiment of the intraocular lens disclosed herein, the intraocular lens further includes a monomer component C as a monomer component that constitutes the copolymer, the monomer component C including a functional group having ultraviolet absorbing ability. The monomer component C is preferably contained in a proportion of at least 0.1 mass% and at most 1.5 mass% relative to the total amount of the acrylate-based monomer component A and the methacrylate-based monomer component B. Such a configuration allows for instance suitably promoting polymerization of the monomer components that make up the copolymer, through application of ultrasonic waves. This is preferable in that a copolymer as a cured body can be obtained quickly, and the intraocular lens can be formed conveniently and in a short time. Such a configuration can be particularly suitable in the production of an intraocular lens in accordance with a cast-mold production method.

In a preferred embodiment of the intraocular lens disclosed herein, the surface portion has a thickness of at least 1 µm and at most 40 µm, the thickness being a dimension in a direction perpendicular to the surface. Such a configuration allows properly reducing the thickness of the surface portion. As a result it becomes possible to suitably reduce the tackiness of the intraocular lens while lowering the proportion of the surface portion of relatively low refractive index.

In a preferred embodiment of the intraocular lens disclosed herein, the intraocular lens is a so-called single-piece intraocular lens including a circular optical portion, and a pair of support portions that support the optical portion, wherein the optical portion and the support portions are formed integrally. Surface tackiness can be kept low even when the intraocular lens disclosed herein is composed of a (meth)acrylate-based copolymer. Even when pressed against the support portion during folding, the support portion can as a result revert smoothly to the original arrangement once the folding pressure is lifted. An intraocular lens is provided as a result that is superior in handleability and operability.

In another aspect, the art disclosed herein provides an intraocular lens insertion system. The intraocular lens insertion system includes any one of the intraocular lenses described above, and an injector, wherein the intraocular lens is accommodated beforehand in the injector. The injector is a dedicated insertion instrument for inserting the intraocular lens into the lens capsule of the eyeball. Such a configuration is preferable since the insertion operation of the intraocular lens at the procedure site can be carried out conveniently, while suppressing contamination of the intraocular lens and curtailing damage at the time of setting of the intraocular lens into the injector.

### Brief Description of Drawings

[Fig. 1]

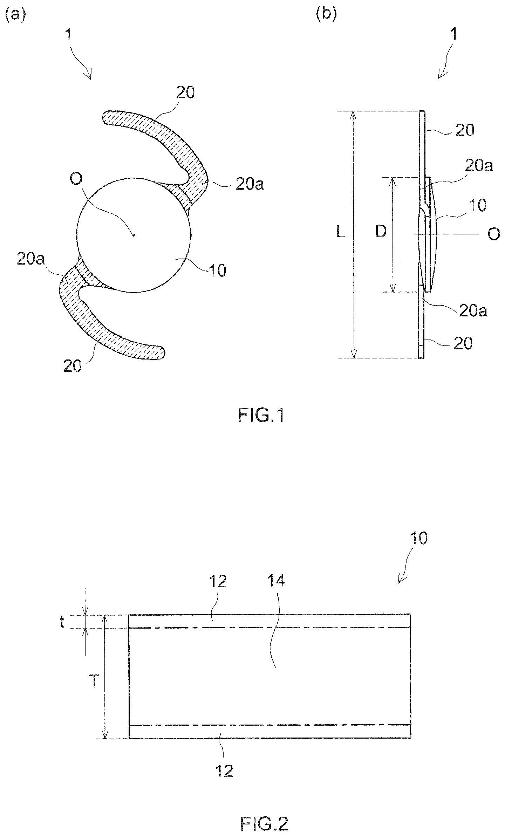

   Fig. 1 is a set of (a) a plan-view diagram and (b) a side-view diagram for explaining schematically the configuration of a single-piece intraocular lens according to an embodiment.
[Fig. 2]
   Fig. 2 is a cross-sectional schematic diagram along the thickness direction of an intraocular lens according to an embodiment.
[Fig. 3]

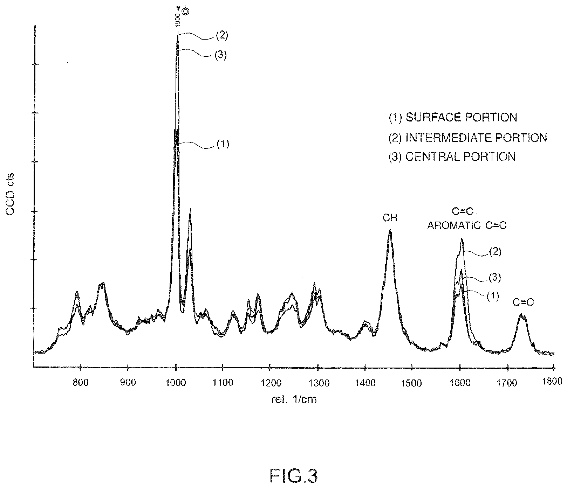

   Fig. 3 is a set of Raman spectra measured at different positions (1) to (3), in the thickness direction, of an intraocular lens according to an embodiment.
[Fig. 4A]

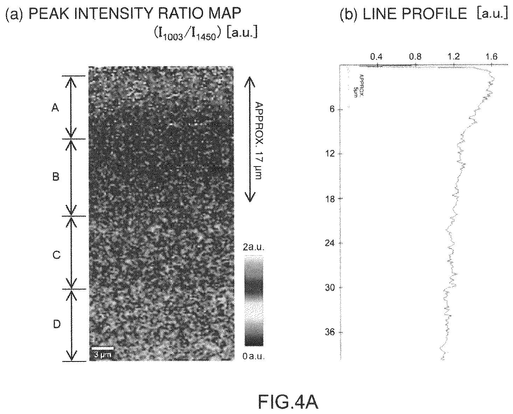

   Fig. 4A is a diagram illustrating an example of (a) a peak intensity ratio map and (b) a line profile along the thickness direction, of a peak intensity ratio I₁₀₀₃/I₁₄₅₀ calculated on the basis of Raman spectra obtained for an intraocular lens according to an embodiment.
[Fig. 4B]

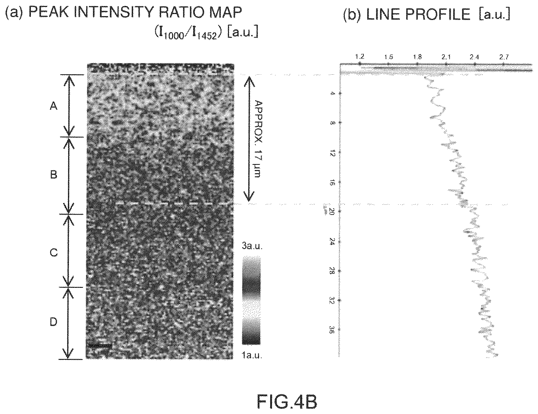

   Fig. 4B is a diagram illustrating an example of (a) a peak intensity ratio map and (b) a line profile along the thickness direction, of a peak intensity ratio I₁₀₀₀/I₁₄₅₂ calculated on the basis of Raman spectra obtained for an intraocular lens according to another embodiment.
[Fig. 5A]

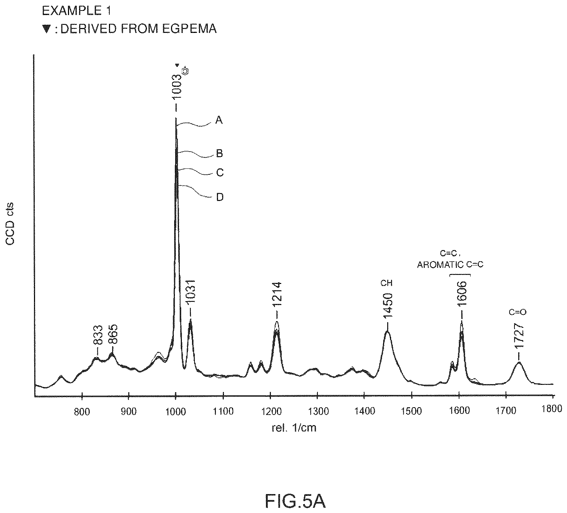

   Fig. 5A is a set of Raman spectra measured at different positions (1) to (4), in the thickness direction, of an intraocular lens according to an embodiment.
[Fig. 5B]

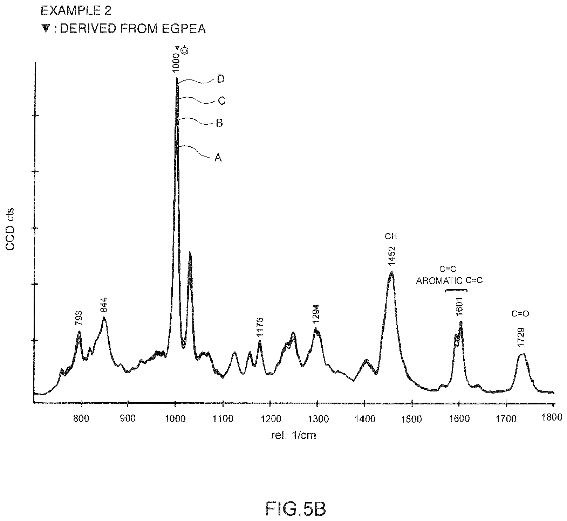

   Fig. 5B is a set of Raman spectra measured at different positions (1) to (4), in the thickness direction, of an intraocular lens according to another embodiment.

### Description of Embodiments

Preferred embodiments of the present invention will be explained next. Any features other than the matter specifically set forth in the present specification and that may be necessary for carrying out the present invention (for example basic structures of intraocular lenses) can be grasped by a person skilled in the art on the basis of the teachings for implementing the invention disclosed in the present specification, and on the basis of common technical knowledge at the time of filing. The invention can be realized on the basis of the disclosure of the present specification and common technical knowledge in the relevant technical field. In the drawings below, members and portions that elicit identical effects are denoted with identical reference symbols, and a recurrent explanation thereof may be omitted or simplified. Unless otherwise stated, a numerical value range notated as "A to B" signifies herein "at least A and at most B".

Fig. 1 is a diagram explaining the configuration of a single-piece intraocular lens according to an embodiment. Herein, (a) in Fig. 1 is a plan-view diagram and (b) is a side-view diagram. Fig. 2 is a diagram illustrating schematically part of a cross section of the intraocular lens in the thickness direction. The intraocular lens 1 is provided with an optical portion 10 having predetermined refractive power, and a pair of support portions 20 for supporting the optical portion 10 within the eye. The optical portion 10 and the support portions 20 are integrally molded out of a same starting resin composition.

The optical portion 10 is for instance a circle in a plan view, having a diameter D of about 5.5 mm to 7 mm, typically of about 6 mm. A direction perpendicular to the diameter D direction of the optical portion 10 is referred to as the thickness direction. The optical portion 10 has, in a side face view, for instance a biconvex lens shape protruding towards both faces (both sides in the thickness direction). The thickness of the optical portion 10 can be determined on the basis of for instance the refractive index of the material that makes up the optical portion 10, and on the basis of the desired refractive power required from the optical portion 10. The thickness of the optical portion 10 is for instance at least 300 µm, and at least 400 µm in an example, and is for instance at most 800 µm, and at most 700 µm in an example, and typically about 600 µm (about ±10%), at a center O of the circular optical portion 10. However, the shape of the optical portion 10 is not limited thereto. The shape of the optical portion 10 may be for instance elliptical or oval, in a plan view. The shape of the optical portion 10 may be for instance a planoconvex lens shape in which, in a side face view, only one surface protrudes and the other surface is flat, or may be for instance a convex meniscus lens shape in which one surface protrudes and the other surface is recessed.

The support portions 20 are formed so as to protrude outward from the peripheral edge of the optical portion 10. The pair of support portions 20 is formed in point contrast with respect to the optical portion 10, taking the center O of the optical portion 10 as an axis of symmetry. One end of each support portion 20 has a loop shape at a free end. Each support portion 20 has, in the vicinity of the connection section with the optical portion 10, a bent portion 20a that is significantly bent within the plane of the lens section. A free end section at each bent portion 20a of the support portions 20 is configured to be further bendable in a direction of drawing close towards the center O. A distance L between the free ends of the pair of support portions 20 is for instance about 11.5 mm to 13.5 mm, and typically about 12 mm to 13 mm. However, the shape of the support portions 20 is not limited thereto.

The optical portion 10 and the support portions 20 of the intraocular lens 1 disclosed herein are both made up of a (meth)acrylate-based polymer of high transparency. The (meth)acrylate-based polymer is a polymer (copolymer) having, as constituent components, a polymerizable acrylate-based monomer component A and a polymerizable methacrylate-based monomer component B. In other words, the (meth)acrylate-based polymer is a copolymer having monomer units corresponding to an acrylate-based monomer component A and monomer units corresponding to a methacrylate-based monomer component B. Generally, the softer a (meth)acrylate-based polymer is, the greater tends to be the tackiness exhibited by the polymer. Among (meth)acrylate-based polymers, for instance homopolymers of acrylate-based monomers tend to have a lower glass transition point (Tg), and are likelier to exhibit tackiness, than homopolymers of methacrylate-based monomers. Accordingly, a characterizing feature of the intraocular lens 1 disclosed herein is that when dividing the intraocular lens 1 into a surface portion 12 which is a region that includes the surface of the intraocular lens 1 in the thickness direction, and a central portion 14 lying closer to the center of the intraocular lens 1 than the surface portion 12 in the thickness direction, as illustrated in Fig. 2, then the surface portion 12 constitutes a methacrylate-rich layer having a higher content of the methacrylate-based monomer component B than the central portion 14.

The monomer units of the methacrylate-based monomer component B need not necessarily be more than the monomer units of the acrylate-based monomer component A in the methacrylate-rich layer that makes up the surface portion 12. It suffices that the content of monomer units of the monomer component B in the methacrylate-rich layer be greater than that at the central portion 14. That is because the tackiness of the (meth)acrylate-based polymer depends also for instance on the structure, for example the number of carbon atoms and the type of functional groups, of the monomer components that make up the polymer.

By adopting thus a configuration wherein the content of methacrylate component in the surface portion 12 is higher than in the central portion 14 it becomes possible to reduce tackiness in the surface portion 12 as compared with that of the central portion 14. At the same time, by adopting a configuration in which the content of acrylate component is higher in the central portion 14 than in the surface portion 12, greater flexibility is achieved in the central portion 14 as compared with that in the surface portion 12. As a result an intraocular lens 1 can be realized overall in which surface tackiness is reduced while the intraocular lens 1 exhibits flexibility.

Alou not categorically so, for instance the peeling adhesion (adhesive strength) of the surface portion 12 can be set to be at most 1 N/cm², since the tackiness of the surface portion 12 depends also on the composition. The peeling adhesion can typically be at most 0.8 N/cm², at most 0.6 N/cm², or for instance at most 0.5 N/cm², or at most 0.4 N/cm², or at most 0.3 N/cm², or at most 0.2 N/cm². The lower limit of the peeling adhesion is not particularly restricted, but can be set for instance to be at least 0.1 N/cm², from the viewpoint of achieving both the flexibility and elastic restoring force required from the intraocular lens 1. The peeling adhesion may be for instance at least 0.3 N/cm², depending on the types of the monomer units that make up the (meth)acrylate-based polymer. The peeling adhesion of the surface of the copolymer that constitutes the intraocular lens 1 can be measured in accordance with the method described in the Examples described below. The tackiness of the surface portion 12 may be reduced to be for instance about at most 90% compared to the tackiness of the central portion 14, and can be set to be at most 80%, typically at most 70%, for instance at most 60%, or at most 50%, and further at most 45%, or at most 40%. The tackiness of the surface portion 12 may be typically at least 20%, for instance at least 30%, compared to the tackiness of the central portion 14.

Such a (meth)acrylate-based polymer can be preferably implemented as a polymer of monomer components that include a polymerizable alkyl (meth)acrylate-based monomer as a main monomer, and further includes, as needed, a secondary monomer that is copolymerizable with the main monomer. The main monomer denotes herein a component that is present at the highest proportion, for instance at least 50 mass%, and typically higher than 50 mass%, among all the monomer components.

An alkyl (meth)acrylate which is a main monomer component can include the acrylate-based monomer component A and the methacrylate-based monomer component B. For instance an alkyl (meth)acrylate represented by the following formula: CH₂ = C(R^{I})COOR^{II} can be suitably used as the acrylate-based monomer component A and the methacrylate-based monomer component B. In the formula above, R^{I} is a hydrogen atom or a methyl group. In the formula above, R^{II} is a linear, branched or cyclic hydrocarbon group. The hydrocarbon group may be an alkyl group, a vinyl group or an aryl group, and may contain an arbitrary functional group or substituent. In a case where R^{II} is a branched or cyclic alkyl group, the number of carbon atoms thereof may be about 1 to 20 (hereafter such a range of number of carbon atoms may be notated as "C₁₋₂₀"). The alkyl group R^{II} is C₁₋₁₄, for instance C₁₋₁₀, typically C₁₋₈ or C₂₋₈, or C₁₋₆, or C₂₋₆, and for instance C₁₋₈ or C₃₋₅, from the viewpoint of imparting flexibility. Specifically, for instance, the alkyl group R^{II} may be methyl (meth)acrylate, ethyl (meth)acrylate, propyl (meth)acrylate, isopropyl (meth)acrylate, n-butyl (meth)acrylate, sec-butyl (meth)acrylate, isobutyl (meth)acrylate, tert-butyl (meth)acrylate, isopentyl (meth)acrylate, sec-pentyl (meth)acrylate, 3-pentyl (meth)acrylate, tert-pentyl (meth)acrylate, hexyl (meth)acrylate, 2-methylbutyl (meth)acrylate, heptyl (meth)acrylate, octyl (meth)acrylate, 2-ethylhexyl (meth)acrylate, nonyl (meth)acrylate, decyl (meth)acrylate, dodecyl (meth)acrylate, stearyl (meth)acrylate, cyclopentyl (meth)acrylate, cyclohexyl (meth)acrylate or the like. Further, R^{II} may be an alkylene group-containing (meth)acrylate having an alkylene group corresponding to the above alkyl group. For instance, the alkyl group may be a substituent or functional group selected from the group consisting of alkylene groups, alkoxy groups, cycloalkyl groups, aryl groups, alkanoyloxy groups, aralkyl groups, acetoxy groups and halogeno groups. As an example, for instance a C₁₋₁₄ or C₁₋₁₀, typically a C₁₋₆ or C₁₋₄ alkylene group may be used as the alkylene group. Specifically, the alkylene group may be a methylene group, an ethylene group, a propylene group, a butylene group, a hexylene group, a heptylene group, an octylene group or dodecylene group, or a functional group including some of the foregoing.

Further, R^{II} may be an alkoxy group-containing (meth)acrylate containing an alkoxy group corresponding to the above alkyl group. The alkoxy group may be for instance a C₁₋₅ or C₁₋₄, typically a C₁ or C₂ alkoxy group. Specifically, the alkoxy group may be a methoxy group, an ethoxy group, a propoxy group, a t-butoxy group, a pentyloxy group or an allyloxy group, or a functional group including some of the foregoing. Examples of such alkoxy group-containing (meth)acrylates include ethylene glycol monomethyl ether (meth)acrylate, ethylene glycol monoethyl ether (meth)acrylate, ethylene glycol monobutyl (meth)acrylate, diethylene glycol monomethyl ether (meth)acrylate, diethylene glycol monoethyl ether (meth)acrylate, diethylene glycol monobutyl ether (meth)acrylate, dipropylene glycol methyl ether (meth)acrylate, dipropylene glycol ethyl ether (meth)acrylate, triethylene glycol monomethyl ether (meth)acrylate, triethylene glycol monoethyl ether (meth)acrylate, tetraethylene glycol monomethyl ether (meth)acrylate, tetraethylene glycol monoethyl ether (meth)acrylate, polyethylene glycol monomethyl ether (meth)acrylate, polyethylene glycol monoethyl ether (meth)acrylate, 2-(2-ethylhexaoxy)ethyl (meth)acrylate, and other alkoxy group-containing (meth)acrylates. Preferred among the foregoing are ethoxy group-containing (meth)acrylates such as ethylene glycol monomethyl ether (meth)acrylate, ethylene glycol monoethyl ether (meth)acrylate, diethylene glycol monomethyl ether (meth)acrylate and diethylene glycol monoethyl ether (meth)acrylate. Any of the foregoing may be used singly or in combinations of two or more types thereof.

The (meth)acrylate-based polymer is preferably a material of high refractive index, in terms of configuring the optical portion 10. From the above viewpoint, for instance R^{II} in the main monomer may be a hydrocarbon group including a cyclic structure, for instance an aromatic ring-containing (meth)acrylate. Examples of aromatic ring-containing (meth)acrylates include aryl (meth)acrylates such as phenyl (meth)acrylate, methoxyphenyl (meth)acrylate and ethoxyphenyl (meth)acrylate; arylalkyl (meth)acrylates such as benzyl (meth)acrylate, 2-phenylethyl (meth)acrylate, 4-phenylbutyl methacrylate and methoxyphenylethyl (meth)acrylate; aryloxyalkyl (meth)acrylates such as phenoxyethyl (meth)acrylate and phenoxypropyl (meth)acrylate; arylalkylene oxide (meth)acrylates such as phenoxydiethylene glycol (meth)acrylate, phenoxy polyethylene glycol (meth)acrylate and phenylphenol ethylene glycol (meth)acrylate. Among the foregoing there can be preferably used benzyl acrylate (BZA), benzyl methacrylate (BZMA), phenylethyl acrylate (PEA), phenylethyl methacrylate (PEMA), ethylene glycol monophenyl ether acrylate (EGPEA) or ethylene glycol monophenyl ether methacrylate (EGPEMA).

The above (meth)acrylates which are main monomers are hydrophobic. Although not particularly limited thereto, therefore, the (meth)acrylate-based polymer may include a secondary monomer component besides the main monomer. As an example, such a secondary monomer component may be a monomer component that imparts hydrophilicity to the (meth)acrylate-based polymer that makes up the intraocular lens 1. The (meth)acrylate-based polymer may contain a monomer that includes a structural unit, typified for instance by hydroxyl groups, that imparts hydrophilicity. For instance a compound copolymerizable with the above main monomers, and which has a hydrophilic group within the monomer structure, can be used herein. Examples of the secondary monomer that imparts hydrophilicity include N-vinyl pyrrolidone, α-methylene-N-methylpyrrolidone and N-vinyl lactams such as N-vinyl caprolactam; hydroxyl group-containing (meth)acrylates such as 2-hydroxyethyl (meth)acrylate, 2-hydroxypropyl (meth)acrylate, 4-hydroxybutyl (meth)acrylate, 6-hydroxyhexyl (meth)acrylate, 2,3-dihydroxypropyl (meth)acrylate and 8-hydroxyoctyl (meth)acrylate; (meth)acrylic acid; and compounds such as (meth)acrylamide, N-methyl (meth)acrylamide, N-ethyl (meth)acrylamide, N-hydroxyethyl (meth)acrylamide, N,N-dimethyl (meth)acrylamide, N,N-diethyl (meth)acrylamide, N-ethylaminoethyl (meth)acrylamide and the like. Preferably used among the foregoing is 2-hydroxyethyl (meth)acrylate or 4-hydroxybutyl (meth)acrylate. Any of the foregoing compounds may be formulated singly or in combinations of two or more types thereof. Preferably, also the secondary monomer component is a (meth)acrylate. Therefore, it is evident that the above main monomer component may have the functional group that imparts hydrophilicity.

Examples of other compounds used in order to configure the (meth)acrylate-based polymer include for instance crosslinking agents (including crosslinking monomers) and polymerization initiators.

The crosslinking agent allows the molecular structure of the obtained (meth)acrylate-based polymer to be made three-dimensional. As a result it becomes possible to increase the mechanical strength and elastic restoring force of the intraocular lens 1, and to stabilize the polymer structure. Examples of the crosslinking agent include hydroxyl group (OH group)-containing monomers, carboxy group-containing monomers, acid anhydride group-containing monomers, amide group-containing monomers, amino group-containing monomers, imide group-containing monomers, epoxy group-containing monomers, (meth)acryloyl morpholine, vinyl ethers and the like. As an example, there can be used specifically a crosslinking agent typified by ethylene glycol di(meth)acrylate, 1,4-butanediol di(meth)acrylate, 1,6-hexanediol di(meth)acrylate, 1,9-nonanediol di(meth)acrylate, trimethylolpropane tri(meth)acrylate and the like. The compounding amount of the crosslinking agent can be established as appropriate on the basis of a relationship with respect to the main monomer. For instance, the compounding amount of the crosslinking agent may be about at least 0.01 mass%, preferably at least about 1 mass%, more preferably at least about 3 mass%, and for instance at least about 4 mass%, with respect to all monomer components. The proportion of the crosslinking agent may be at most 10 mass%, more preferably at most 8 mass%, and for instance at most 5 mass%.

An ultraviolet-absorbing monomer component C having ultraviolet absorbing ability can be incorporated as the secondary monomer component. The crystalline lens has the property of not readily transmitting ultraviolet rays, whereas intraocular lenses can let ultraviolet rays through, which poses a risk of damage to the retina. Therefore, ultraviolet absorbing ability can be imparted to the (meth)acrylate-based polymer that makes up the intraocular lens 1, by incorporating the ultraviolet-absorbing monomer component C having ultraviolet absorbing ability, as the secondary monomer component. This allows suppressing damage to the retina, and lowering the risk of eye diseases derived from ultraviolet rays such as macular degeneration and keratitis. The term "ultraviolet-absorbing monomer" refers to monomers in general having a functional group that exhibits ultraviolet absorbing ability. The term functional group having ultraviolet absorbing ability refers to a set of groups of atoms having an absorption spectrum in the ultraviolet region. Generic instances of such functional groups having ultraviolet absorbing ability include alkyl residues, carboxylic acid residues, alcohol residues, amino residues, acyl groups and the like of ultraviolet absorbing compounds that are widely used as ultraviolet absorbers. As an example, the ultraviolet absorbing functional group encompasses broadly for instance a group of atoms resulting from excluding hydrogen atoms from carboxyl groups, hydroxyl groups, amino groups and the like in ultraviolet absorbing compounds, and acyl groups in ultraviolet absorbing compounds. More specifically, a benzotriazole-based monomer component, a benzophenone-based monomer component, a salicylic acid-based monomer component and a cyanoacrylate-based monomer component can be preferably used as the ultraviolet-absorbing monomer component C.

It is deemed that a content of about 0.1 to 1.5 mass% of the ultraviolet-absorbing monomer component C with respect to the total amount of the acrylate-based monomer component A and the methacrylate-based monomer component B that are the main monomers, is appropriate herein. That is because when the proportion of the monomer component C is excessively lower than 0.1 mass%, the ultraviolet absorbing ability of the intraocular lens 1 decreases excessively, and the functionality of the intraocular lens 1 may fail to be effectively brought out. When the proportion of the monomer component C is excessively higher than 1.5 mass%, the total quantity of ultraviolet rays passing through the intraocular lens 1 becomes excessively low, which may result in darkening of the field of view. The proportion of the ultraviolet-absorbing monomer component C is typically about 0.1 to 1.2 mass%, and more preferably for instance about 0.2 to 1 mass%.

The crystalline lens has a yellowish coloration, and accordingly has also the property of suppressing some transmission of blue light, which is the complementary color. Therefore, color vision is preferably adjusted by imparting coloring to the (meth)acrylate-based polymer disclosed herein, for instance using a yellow dye or a red dye. As the colorant there can be used a known azo-based compound or pyrazole-based compound utilized as a colorant in this kind of intraocular lenses. The compounding amount of the colorant can be established as appropriate on the basis of a relationship with respect to the main monomer. For instance, the content of the colorant preferably lies in the range of about 0.01 to 1 mass% relative to all monomer components.

For instance a radical polymerization initiator made up of a benzoin ether or an amine can be preferably used as the polymerization initiator. As a specific example there can be used a polymerization initiator typified by 2,2-azobisisobutyronitrile, azobis dimethylvaleronitrile, benzoin, methyl ortho-benzoyl benzoate or the like. The compounding amount of the polymerization initiator can be established as appropriate on the basis of a relationship with respect to the main monomer. For instance, the content of the polymerization initiator preferably lies in the range of about 0.001 to 0.1 mass%, for instance 0.01 to 1 mass%, relative to all monomer components.

The above intraocular lens 1 is required to boast very high transparency, refractive index, and also flexibility. Therefore, it is preferable to keep as low as possible the content of components other than the acrylate-based monomer component A and the methacrylate-based monomer component B that make up the (meth)acrylate-based polymer, the hydroxy group-containing monomer that imparts hydrophilicity, and the crosslinking agent. For instance, the total content of components other than the acrylate-based monomer component A and the methacrylate-based monomer component B, the hydroxy group-containing monomer that imparts hydrophilicity, and the crosslinking agent, can be suitably set to about 5 mass% or less with respect to 100 mass% as the total amount of polymerizable monomer components. The above other components may be for instance 4 mass% or less, or 3 mass% or less.

The intraocular lens 1 can be obtained through copolymerization of a starting material composition that contains the above monomer components and other compounds. For the polymer of the starting material composition various monomer materials are selected, and the formulation of the whole is adjusted as appropriate so that the polymer has the characteristic of being easily bendable, while not taking water up at room temperature. The surface of the (meth)acrylate-based polymer obtained upon copolymerization of the starting material composition on the basis of a conventional method may exhibit comparatively high tackiness at normal temperature. In the art disclosed herein, by contrast, the intraocular lens 1 is produced by resorting to a so-called cast-mold production method, and the surface portion 12 is polymerized in an oxygen supply environment.

In a cast-mold production method, specifically, a casting mold having a cavity (void) corresponding to the shape of the target intraocular lens 1 is prepared, a starting material composition is supplied into the casting mold, and the starting material composition is polymerized within the casting mold. In the art disclosed herein, the casting mold is made up of an oxygen-permeable material, to thereby create a state in which the methacrylate-based monomer component B within the starting material composition is relatively abundant at the surface portion 12 in contact with the casting mold, and to promote polymerization. A glass material or SUS steel material commonly used in conventional art are not appropriate herein as the material that makes up the casting mold. Although not limited thereto, as a preferred example there is used an organic material that exhibits comparatively high oxygen permeability, for instance a polyether ether ketone (PEEK) resin. As a result, this allows building a methacrylate-rich layer having a higher content of methacrylate-based monomer component B as the monomer component, at the surface portion 12, as compared with the central portion 14, and allows achieving an intraocular lens 1 in which surface tackiness is suitably reduced.

The combination of monomer components is important in the production of the intraocular lens 1 disclosed herein from the viewpoint of suitably forming the methacrylate-rich layer on the surface portion 12 of the intraocular lens 1. In a preferred embodiment, the acrylate-based monomer component A may include an alkoxy group-containing acrylate, the methacrylate-based monomer component B may include an aromatic ring-containing methacrylate, and there may be further included a hydrophilic hydroxy group-containing acrylate, as another monomer component. Such a monomer combination allows for instance providing a methacrylate-rich layer of comparatively high hardness on the surface portion 12 of the intraocular lens 1, and reducing the tackiness of the surface of the intraocular lens 1. Moreover it becomes possible to soften the central portion 14 of the intraocular lens 1, and to realize an intraocular lens 1 that exhibits both adequate flexibility and shape recovery. Herein, (meth)acrylate-based polymers having such a configuration have exhibited a tradeoff in that suppression of tackiness entails a likelier occurrence of glistenings. In the art disclosed herein, however, the above acrylate-based monomer component A is prescribed to include an alkoxy group-containing acrylate, not an alkyl group-containing acrylate. Adequate hydrophilicity can be imparted to a hydrophobic (meth)acrylate-based polymer, while suitably suppressing for instance the occurrence of glistenings, by adopting thus a combination of an alkoxy group-containing acrylate, a methacrylate-based monomer component B and a hydroxy group-containing acrylate. Such features are deemed to be those of a hitherto unknown novel composition.

Preferably, the alkoxy group-containing acrylate includes a compound represented by General formula (1) below. In the formula, R1 is a methyl group or ethyl group, and n is an integer from 1 to 4. Examples of such compounds include ethoxy group monomethyl ether acrylates containing 1 to 4 ethoxy groups and ethoxy group monoethyl ether acrylates containing 1 to 4 ethoxy groups. Preferably used among the foregoing are ethylene glycol monomethyl ether acrylate (2-methoxyethyl acrylate: MEA), ethylene glycol monoethyl ether acrylate (2-ethoxyethyl acrylate: EEA), diethylene glycol monomethyl ether acrylate (2-(2-ethoxyethoxy) methyl acrylate: EEMA) or diethylene glycol monoethyl ether acrylate (2-(2-ethoxyethoxy)emethyl acrylate: EEEA).

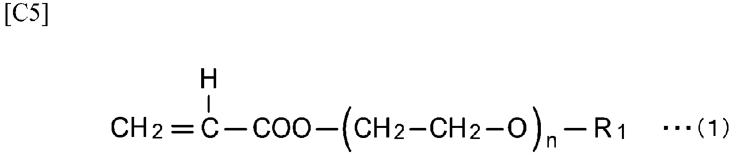

Preferably, the aromatic ring-containing methacrylate includes a compound represented by General formula (2) below. In the formula, R2 is a C₁₋₈ linear or branched alkylene group, and X is absent or represents an oxygen atom. Examples of such a compound include benzyl methacrylate (BZMA), phenylethyl methacrylate (PEMA) and ethylene glycol monophenyl ether methacrylate (EGPEMA). Benzyl methacrylate is preferably utilized among the foregoing.

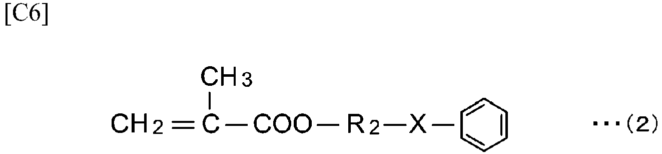

Preferably, the hydroxy group-containing acrylate includes a compound represented by the following formula: CH₂ = CH-COO-R_{H}-OH. In the formula, R_{H} is a C₁₋₈ linear or branched alkylene group. Examples of such a compound include 2-hydroxyethyl acrylate, 2-hydroxypropyl acrylate, 4-hydroxybutyl acrylate, 6-hydroxyhexyl acrylate, 2,3-dihydroxypropyl acrylate and 8-hydroxyoctyl acrylate. Among the foregoing 2-hydroxyethyl acrylate (HEA) or 4-hydroxybutyl acrylate (HBA) is preferably used.

When as described above the acrylate-based monomer component A does not include a compound having an aromatic ring and the methacrylate-based monomer component B includes a compound having an aromatic ring, for instance Raman spectroscopic analysis allows checking whether or not the surface portion 12 has a higher content of the monomer component B, as a monomer component, than the central portion 14. For instance in a Raman spectroscopic analysis spectrum with excitation wavelength at 532 nm, a Raman peak derived from the aromatic ring in the methacrylate-based monomer component B is observed in the vicinity of 1003 cm⁻¹. When for instance the acrylate-based monomer component A includes a compound having a C-H bond of an alkyl group, alkoxy group or the like in the structure, the Raman peak derived from the C-H bond is observed in the vicinity of 1450 cm⁻¹.

Such being the case, it is possible to ascertain for instance which region has the higher proportion of the methacrylate-based monomer component B in a copolymer of a (meth)acrylate-based monomer and a hydroxy group-containing monomer, represented by General formulas (1) and (2) above, by calculating a ratio R (I₁₀₀₀/I₁₄₅₂) of the intensity I₁₀₀₀ of a Raman peak observed in the vicinity of 1000 cm⁻¹ with respect to the intensity I₁₄₅₂ of a Raman peak observed in the vicinity of 1452 cm⁻¹, for the surface portion 12 and the central portion 14, and by comparing then the calculated ratios. For instance in a comparison of a peak intensity ratio Rs of the surface portion 12 of the intraocular lens 1 and a peak intensity ratio Rc of the central portion 14, it can be verified that the proportion of the methacrylate-based monomer component B is higher in the surface portion 12 than in the central portion 14 if the relationship Rs>Rc is satisfied. In other words, it can be verified that the proportion of the methacrylate-based monomer component B in the surface portion 12 is higher than in the central portion 14. Preferably, the peak intensity ratios Rs, Rc of the surface portion 12 and the central portion 14 satisfy the relationship Rs ≥ 1.1 × Rc, and more preferably satisfy the relationship Rs ≥ 1.2 × Rc or Rs ≥ 1.3 × Rc.

Preferably, the acrylate-based monomer is relatively more abundant in a proportion of the total of acrylate-based monomers resulting from combining the acrylate-based monomer component A and the hydroxy group-containing acrylate, and the methacrylate-based monomer component B, from among the monomer components that make up the (meth)acrylate-based polymer. As a result a (meth)acrylate-based polymer can be obtained that is overall flexible, but without making the intraocular lens excessively hard. In further detail, preferably, the aromatic ring-containing methacrylate is at least 40 mass% and at most 52 mass%, the alkoxy group-containing acrylate is at least 35 mass% and at most 46 mass%, and the hydroxy group-containing acrylate is at least 8 mass% and at most 12 mass%. Although not limited thereto, in this case the total of the aromatic ring-containing methacrylate, the alkoxy group-containing acrylate and the hydroxy group-containing acrylate is at least 90 mass%, and more preferably at least 95 mass%.

From another standpoint, the (meth)acrylate-based polymer is preferably a material of high refractive index, in terms of configuring the optical portion 10. Front this viewpoint, for instance R² in the main monomer may include a hydrocarbon group having a cyclic structure, for instance an aromatic ring. A comparison between the flexibility of acrylate-based monomers and methacrylate-based monomers reveals that acrylate-based monomers can yield polymers of higher flexibility.

Therefore, for instance the acrylate-based monomer component A preferably includes a compound represented by General formula (3) below. In the formula, R3 is a C₁₋₆ linear or branched alkylene group. Specific examples of the alkylene group include for instance methylene groups, ethylene groups, propylene groups, butylene groups and hexylene groups. Further, Y represents absence of an element, or an oxygen atom. Examples of such compounds include benzyl acrylate and polyethylene glycol monophenyl ether acrylate.

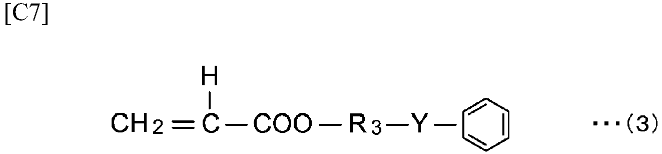

In a case where as described above the acrylate-based monomer component A includes a cyclic hydrocarbon group, then R^{II} in the methacrylate-based monomer component B is preferably for instance a C₃₋₅ linear alkyl group. Such a monomer component B is preferred by virtue of having the effect of increasing cured product transparency. For instance the methacrylate-based monomer component B includes preferably a butyl methacrylate represented by General formula (4) below. When the acrylate-based monomer component A has a benzene ring structure that may give rise to steric hindrance, for instance the monomer component B can move easily to the surface portion 12, and the proportion of the monomer component B in the surface portion 12 can be increased, by virtue of the fact that the methacrylate-based monomer component B has a linear structure with comparatively few carbon atoms.

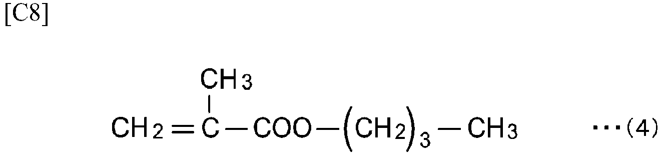

When as described above the acrylate-based monomer component A includes a compound having an aromatic ring (phenyl group) and the methacrylate-based monomer component B does not include a compound having an aromatic ring, for instance Raman spectroscopic analysis allows checking whether or not the surface portion 12 has a greater content of the monomer component B, as a monomer component, than the central portion 14. For instance in a Raman spectroscopic analysis spectrum with excitation wavelength at 532 nm, a Raman peak derived from an aromatic ring included in the acrylate-based monomer component A is observed in the vicinity of 1000 cm⁻¹. When the methacrylate-based monomer component B includes a compound having for instance a C-H bond of an alkyl group in the structure, a Raman peak derived from the C-H bond of the alkyl group is observed in the vicinity of 1452 cm⁻¹.

Such being the case, it is possible to ascertain which region has the higher proportion of the acrylate-based monomer component A, for instance in a copolymer of (meth)acrylate-based monomers represented by General formulas (3) and (4) above, by calculating a ratio R (= I₁₀₀₀/I₁₄₅₂) of the intensity I₁₀₀₀ of a Raman peak observed in the vicinity of 1000 cm⁻¹ with respect to the intensity I₁₄₅₂ of a Raman peak observed in the vicinity of 1452 cm⁻¹, for the surface portion 12 and the central portion 14, and by comparing then the calculated ratios. For instance upon comparison of the peak intensity ratio Rs of the surface portion 12 and the peak intensity ratio Rc of the central portion 14 of the intraocular lens 1, it can be verified that the proportion of the acrylate-based monomer component A is higher in the central portion 14 than in the surface portion 12, if the relationship Rs<Rc is satisfied. In other words it can be verified that the proportion of the methacrylate-based monomer component B in the surface portion 12 is higher than in the central portion 14. Preferably, the peak intensity ratios Rs, Rc of the surface portion 12 and the central portion 14 satisfy the relationship Rs ≤ 0.95 × Rc and more preferably satisfy the relationship Rs ≤ 0.9 × Rc.

Preferably, the acrylate-based monomer component A is relatively more abundant in the proportion of the acrylate-based monomer component A and the methacrylate-based monomer component B, from among the monomer components that make up the (meth)acrylate-based polymer. As a result it becomes possible to obtain a flexible (meth)acrylate-based polymer of high refractive index. The high photopolymerizability of the acrylate-based monomer component can be exploited at the time of polymerization. Taking the totality of the monomer components as 100 mass%, the proportion of the acrylate-based monomer component A and the methacrylate-based monomer component B is such that the methacrylate-based monomer component B is at least about 30 mass%, preferably at least 35 mass%, and preferably for instance about 40 mass%, when the acrylate-based monomer component A is 50 mass%. When the acrylate-based monomer component A is set to 50 mass%, preferably the methacrylate-based monomer component B is set to be at most about 50 mass%, and preferably at most 45 mass%.

Among the monomer components that make up the (meth)acrylate-based polymer, the proportion of the acrylate-based monomer component A relative to all monomer components is preferably at least 42 mass% and at most 62 mass%. The proportion of the methacrylate-based monomer component B in all monomer components is preferably at least 35 mass% and at most 55 mass%. For instance the main monomers are, as a total, preferably at least 50 mass%, preferably at least 75 mass%, and more preferably for instance at least 77 mass%. The main monomer component is not limited thereto, and can be set to be, as a total, at most about 97 mass%.

In the copolymer of (meth)acrylate-based monomers represented by Formulas (3) and (4), the refractive index of the surface portion 12 can be reduced with respect to that of the central portion 14 by virtue of the fact that the surface portion 12 has a greater content of methacrylate component than the central portion 14, as described above. Although not categorically so, for instance the refractive index of aromatic acrylate takes on a value of about 1.5180 which is higher than that of a non-aromatic ring-containing acrylate, since the refractive index of the surface portion 12 depends also for instance on the composition thereof. By contrast, the refractive index of general alkyl methacrylates is relatively low, of about 1.4230. Accordingly, a configuration wherein the surface portion 12 of the intraocular lens 1 has a higher content of methacrylate component than the central portion 14 can be ascertained for instance by examining refractive indices. As an example, the refractive index of the surface portion 12 of the intraocular lens 1 disclosed herein can be at least 1.505 and less than 1.51, and the refractive index of the central portion 14 can be at least 1.51 and at most 1.52. More specifically, the intraocular lens 1 can be provided as an intraocular lens in which the refractive index of the surface portion 12 is reduced to about 1.5080±0.0020 when the refractive index of the central portion 14 is set to about 1.5185±0.0020. Accordingly, the refractive index of the surface portion 12 may be set to be lower than that the refractive index of the central portion 14 by at least about 0.002, or by at least about 0.005, for instance by at least about 0.008, and further by at least about 0.010. It can be appropriate herein to set the difference between the refractive indices of the surface portion 12 and the central portion 14 to be for instance at most about 0.020. The refractive index of the intraocular lens 1 is not limited thereto, and may be for instance at least about 1.50 and at most 1.53.

The surface tackiness of such an intraocular lens 1 is reduced by controlling the manner in which the intraocular lens 1 is polymerized, and adjusting the components that make up the surface portion 12. As a result, an intraocular lens 1 can be realized such that the surface portion 12 has a comparatively small surface roughness Ra. For instance the surface roughness of the intraocular lens 1 can be set to at most 0.35 nm, and may be set to at most 0.3 nm, for instance at most 0.25 nm, or at most 0.20 nm.

As described above, the surface portion 12 and the central portion 14 are formed integrally with each other, without bonding of any other separately formed member. Therefore, the proportion of the acrylate-based monomer component A and the methacrylate-based monomer component B in the surface portion 12 and in the central portion 14 changes continuously in the thickness direction. In a typical example, the composition of the surface portion 12 changes continuously in such a manner that the acrylate-based monomer component A increases and the methacrylate-based monomer component B decreases, in the thickness direction, with increasing distance from the outermost surface. It is found that the central portion 14 has a roughly uniform composition, since the influence of the casting mold no longer reaches the central portion 14. It is thus found that in one embodiment of the intraocular lens 1 disclosed herein, surface tackiness is suitably reduced by virtue of the fact that a gradient composition is realized in the surface portion 12.

Although not particularly limited thereto, the thickness of the surface portion 12 can be set to be for instance at least 1 µm and at most 40 µm. Preferably, the thickness of the surface portion 12 is prescribed to be for instance at least 1 µm, as a result of which characteristics such as tackiness and refractive index can be caused to vary significantly with respect to those of the central portion 14. The thickness of the surface portion 12 is typically at least 5 µm, and may be for instance at least 10 µm, or be set to be at least 15 µm. An excessive thickness of the thickness of the surface portion 12 is undesirable, since in that case the proportion of the surface portion 12 having a relatively low refractive index increases excessively, and the refractive power of the optical portion 10 decreases. From the above standpoint, the thickness of the surface portion 12 may be for instance at most 35 µm, and can be set to at most 30 µm; more suitably, the thickness of the surface portion 12 is at most 25 µm.

The intraocular lens 1 disclosed herein is composed of a (meth)acrylate-based polymer, and accordingly the intraocular lens 1 suitably exhibits foldable flexibility and an elastic restoring force similar to those of conventional intraocular lenses. The surface tackiness of the intraocular lens 1 disclosed herein is reduced. Thus even when folded to smaller size, therefore, the intraocular lens 1 unfolds as soon as the folding force no longer acts, and reverts to the original shape. As a result the intraocular lens 1 disclosed herein can be suitably used in particular as an intraocular lens of a form where the intraocular lens 1 is bent to a smaller size, by a dedicated insertion instrument, and is then inserted into the eye. From the above viewpoint, the art disclosed herein can preferably provide this intraocular lens insertion system.

Although specifically illustration is omitted, the intraocular lens insertion system has the above-described intraocular lens 1 and an injector. The injector has for instance a syringe-type injector body and a plunger. The injector body is configured so that the width of an internal space thereof decreases at the tip side, such that the intraocular lens 1 becomes folded to a smaller size, in the width direction, as the intraocular lens 1 passes through this internal space. The intraocular lens 1 is set beforehand, in a flat state, in the interior of the injector body. To insert the intraocular lens 1 for instance into the capsule, for example a lubricant made up of a viscoelastic substance or the like is supplied into the injector body, and thereafter the plunger in pushed into the injector body. As a result, the intraocular lens 1 becomes bent so as to fold up, and is then ejected outward through a discharge tip of the injector, together with the lubricant. In consequence, the intraocular lens 1 can be inserted conveniently into the lens capsule of the eyeball. For instance the injector may be made up of only a cartridge unit which is a portion, within the injector body. From an accommodating section in which the intraocular lens 1 is accommodated up to an outlet through which the intraocular lens 1 is ejected. The cartridge unit may be configured for instance to be attachable/detachable to/from the injector body.

### Examples

Several Examples pertaining to the present invention will be explained next. However, the present invention is not meant to be limited to the concrete examples illustrated herein.

### (Test example 1)

### [Preparation of an intraocular lens]

A starting material composition for intraocular lens of Example 1 was prepared by using 36 parts by mass of 2-methoxyethyl acrylate (MEA) as the acrylate-based monomer component A, 50 parts by mass of ethylene glycol monophenyl ether methacrylate (EGPEMA) as the methacrylate-based monomer component B, as well as 10 parts by mass of 4-hydroxybutyl acrylate (HBA) and 4 parts by mass of 1,4-butanediol diacrylate (BDDA), and by further adding thereto an ultraviolet absorber and a polymerization initiator, with uniform mixing of the whole.

A starting material composition for intraocular lens of Example 2 was prepared by using 50 parts by mass of ethylene glycol monophenyl ether acrylate (EGPEA) as the acrylate-based monomer component, 37 parts by mass of n-butyl methacrylate (BMA) as the methacrylate-based monomer component, and also a total of 13 parts by mass of methyl methacrylate (MMA), n-butyl acrylate (BA) and 1,4-butanediol diacrylate (BDDA), and by further adding thereto an ultraviolet absorber and a polymerization initiator, with uniform mixing of the whole.

Each starting material composition was injected into a casting mold made of an organic compound (PEEK) and provided with a pier, for a biconvex lens shape having a diameter 6 mm and a thickness of 0.56 mm, and thermal polymerization was carried out within an air oven, at conditions of 60°C for 12 hours, and 100°C for 12 hours, to thereby prepare a plurality of a lens molded bodies of Example 1 and Example 2.

### [Raman spectroscopic analysis 1 of a lens molded body]

The lens molded body of Example 2 was assessed first for the concentration of EGPEA used as one of the main monomers, along the thickness direction of the lens molded body up to the central portion. Specifically, each prepared lens molded body was cut in the radial direction of a circular lens optical portion, using a microtome in a freezing environment, to thereby prepare measurement cross sections (slices) of the lens molded body. Then Raman spectroscopic analysis was carried out on the three measurement points below, along the thickness direction, at the center of the circle of the lens optical portion, within the measurement cross section.
(1) surface portion: point at about 2 µm from the outermost surface
(2) intermediate portion: point being a midpoint between the surface portion and a below-described central portion, at about 140 µm from the outermost surface
(3) central portion: point being the center in the thickness direction, at about 280 µm from the outermost surface.

Raman spectroscopic analysis was carried out under the measurement conditions below using a SNOM/AFM/Raman combined analyzer (ALPHA300RSA) by WITec K.K.. Fig. 3 illustrates the Raman spectra obtained as a result. In Fig. 3, the Raman spectra obtained for the three measurement points and normalized with respect to the peak intensity in the vicinity of 1452 cm⁻¹, attributed to the CH antisymmetric vibration of an alkyl group, are extracted only for a range of about 800 to 1800 cm⁻¹, and are depicted superimposed on one another.

### [Raman microscopy analysis conditions]

Excitation wavelength: 532 nm
Measurement wavenumber range: about 125 to 3800 cm⁻¹
Grating: 600 gr/mm
Objective lens: 100×
Measurement time: 0.5 sec/spectrum
Detector: EMCCD

It could be observed that in the Raman spectra obtained for the three measurement points, the position, shape, height and so forth of the detection peaks matched substantially over the entire measurement range. However, variability was observed in some peak heights, for instance in the vicinity of 793 cm⁻¹, 1000 cm⁻¹, 1030 cm⁻¹, 1176 cm⁻¹, 1294 cm⁻¹ and 1601 cm⁻¹, as illustrated in Fig. 3. These peaks exhibit collectively about the same peak height for (2) the intermediate portion and (3) the central portion, whereas the peaks of the (1) surface portion are lower. The peak at 1000 cm⁻¹ is attributed to an aromatic carbon ring, and the peak at 1601 cm⁻¹ is attributed to C = C in the skeleton and an aromatic carbon ring. Accordingly, it is deemed that the variability between the peak heights depends also on differences in the abundance ratio of EGPEA.

A calculation of a ratio I₁₀₀₀/I₁₄₅₂ of the peak intensity I₁₀₀₀ at 1000 cm⁻¹ with respect to the peak intensity I₁₄₅₂ at 1452 cm⁻¹, on the basis of the Raman spectra at the (1) surface portion, (2) intermediate portion and (3) central portion, revealed that the ratio was about 1.76 at the (1) surface portion, about 2.54 at the (2) intermediate portion and about 2.20 at the (3) central portion. From this it was found that the proportion of EGPEA was significantly lower (for instance 0.8 times lower) at the (1) surface portion than at the (2) intermediate portion and the (3) central portion of the lens molded body. In other words, it was found that the proportion of methacrylate component was higher at the (1) surface portion than at the (2) intermediate portion and at the (3) central portion of the lens molded body.

### [Raman spectroscopic analysis 2 of a lens molded body]

The concentration distribution in the thickness-direction of EGPEMA or EGPEA used as one main monomer was assessed next for part of a cross-sectional region of the lens molded bodies of Example 1 and Example 2. Specifically, measurement cross sections (slices) of the lens molded bodies were prepared in the same way as in Raman spectroscopic analysis 1 above. A region of about 20 µm in the surface direction × about 40 µm in the thickness direction was scanned with excitation light from the surface (point about 2 µm from the outermost surface), while encompassing the center of the circle of the lens optical portion, within the measurement cross section, to perform a Raman spectroscopic analysis (Raman mapping analysis). The same SNOM/AFM/Raman combined analyzer as in Test example 1 was used herein for Raman spectroscopic analysis, under the analysis conditions below.

### [Raman mapping analysis conditions]

Excitation wavelength: 532 nm
Measurement wavenumber range: about 125 to 3800 cm⁻¹
Grating: 600 gr/mm
Objective lens: 100×
Measurement range: 20 µm × 40 µm
Measurement points: 100 points × 200 points
Measurement time: 0.2 sec/spectrum (mapping)
Detector: EMCCD

Respective ratios of the height I_{CH} of a peak derived from CH in the vicinity of 1450 to 1452 cm⁻¹ and a height I_{Arm} of a peak derived from an aromatic ring in the vicinity of 1000 to 1003 cm⁻¹ were calculated on the basis of the result of Raman spectroscopic analysis of the lens molded bodies of Example 1 and Example 2, and are depicted as (a) in Figs. 4A, 4B, as a map of peak intensity ratio I_{Arm}/I_{CH} (specifically Example 1: I₁₀₀₃/I₁₄₅₀ and Example 2: ratio I₁₀₀₀/I₁₄₅₂). A line profile of the ratio I_{Arm}/I_{CH} based on a Raman spectroscopic analysis pertaining to arbitrary scanning performed along the thickness direction is depicted in (b) of Figs. 4A, 4B.

It was found that although details in the peak intensity ratio I_{Arm}/I_{CH} map (a) are difficult to read on account of the grayscale display, the ratio I_{Arm}/I_{CH} took on a substantially uniform value in the surface direction. It was also found that the ratio I_{Arm}/I_{CH} took on a gradually lower value in the lens molded body of Example 1, and a gradually higher value in the lens molded body of Example 2, with increasing distance from the surface along the thickness direction. In other words, the compositions of the lens molded bodies in Example 1 and Example 2 were both substantially uniform in the surface direction parallel to the surface of the lens molded body, whereas in the thickness direction perpendicular to the surface direction, the methacrylate component was rich in the surface portion, with the proportion of the methacrylate component dropping and the proportion of the acrylate component rising with increasing distance from the surface. Herein (a) and (b) in Figs. 4A, 4B indicate that the peak intensity ratio in a region relatively close to the surface fluctuates significantly in the lens molded body of Example 1, while the fluctuation in peak intensity ratio is relatively gentle, in the lens molded body of Example 2.

### [Raman spectroscopy analysis 3 of a lens molded body]

The change in the abundance ratio of EGPEMA or EGPEA used as one main monomer, in the lens molded bodies of Example 1 and Example 2, was checked in more detail within a Raman mapping analysis region. Specifically, measurement cross sections (slices) of the lens molded bodies of the examples were prepared, the four regions below were set from the surface in the vicinity of the center of a circular lens optical portion, within the measurement cross section, and a Raman spectroscopic analysis was carried out for each of the regions. The conditions of the Raman spectroscopic analysis were identical to those of analysis 1 above. The Raman spectra obtained as a result for the lens molded bodies of Example 1 and Example 2 are depicted in Figs. 5A and 5B, respectively. The Raman spectra obtained for the four measurement points below were extracted for a range of about 800 to 1800 cm⁻¹, were normalized with respect to the peak intensity I_{CH} in the vicinity of 1450 cm⁻¹ (Example 1) and in the vicinity of 1452 cm⁻¹ (Example 2), attributed to CH antisymmetric vibration in alkyl groups, and were displayed superimposed on each other.
Region 1: range of about 0 to 10 µm from the surface (measurement point about 2 µm)
Region 2: range of about 10 to 20 µm from the surface (measurement point about 10 µm)
Region 3: range of about 20 to 30 µm from the surface (measurement point about 20 µm)
Region 4: range of about 30 to 40 µm from the surface (measurement point about 30 µm)

As Figs. 5A, 5B reveal, it was observed that the position, shape and so forth of the detection peaks of the Raman spectra obtained for each of the measurement points roughly matched each other, over the entire measurement range. In a detailed observation, however, it was found for instance that for the lens molded body of Example 1 in Fig. 5A the peak derived from a C = O bond in the vicinity of 1727 cm⁻¹ exhibited substantially the same height in measurement points (1) to (4), whereas the height of the peak in the vicinity of 1003 cm⁻¹, derived from stretching vibration of an aromatic carbon ring and the height of the peak in the vicinity of 1606 cm⁻¹ derived from a C = C bond in the aromatic ring and the skeleton, varied significantly. Similarly, it was found for instance that for the lens molded body of Example 2 in Fig. 5B the peak derived from a C = O bond in the vicinity of 1729 cm⁻¹ exhibited substantially the same height in measurement points (1) to (4), whereas the height of the peak in the vicinity of 1000 cm⁻¹, derived from stretching vibration of an aromatic carbon ring and the height of the peak in the vicinity of 1601 cm⁻¹ derived from a C = C bond in the aromatic ring and the skeleton, varied significantly. It is deemed that the above peak height variability can be ascribed to differences in the abundance ratio of EGPEMA, used as the methacrylate-based monomer component B, at the respective measurement points, in the lens molded body of Example 1 and can be ascribed to differences in the abundance ratio of EGPEA used as the acrylate-based monomer component A, at the respective measurement points, in the lens molded body of Example 2. Accordingly there was calculated a ratio I_{Arm}/I_{CH} of the height of the peak in the vicinity of 1450 cm⁻¹ derived from CH and the height of the peak in the vicinity of 1000 cm⁻¹ derived from an aromatic ring; the results are given in Table 1.

### [Table 1]

**Table 1**

| | | Peak intensity | | |
|---|---|---|---|---|
| Example | Measurement point | Aromatic carbon ring (around 1000 cm⁻¹) | CH bond (around 1450 cm⁻¹) | Peak intensity ratio I_{Arm}/I_{CH} |
| 1 | 1 | 5606.6 | 3740.7 | 1.50 |
| | 2 | 4920.2 | 3922.6 | 1.25 |
| | 3 | 4334.4 | 3752.2 | 1.16 |
| | 4 | 3958.7 | 3617.6 | 1.09 |
| 2 | 1 | 2012.5 | 3760.8 | 0.54 |
| | 2 | 2912.7 | 4690.6 | 0.62 |
| | 3 | 3019.6 | 4379.6 | 0.69 |
| | 4 | 2967.9 | 4107.0 | 0.72 |

As Table 1 shows, it was found that in the lens molded body of Example 1 the proportion of EGPEMA used as the methacrylate-based monomer component B was relatively high at measurement point (1) close to the surface, but decreased gradually towards measurement point (4), which is the center side in the thickness direction. In the lens molded body of Example 2, conversely, it was found that the proportion of EGPEA used as the acrylate-based monomer component A was relatively low at measurement point (1) close to the surface, but increased gradually towards measurement point (4), which is the center side in the thickness direction. It was thus verified that the ratio I_{Arm}/I_{CH} corresponds well to the (b) line profile in Figs. 4A, 4B.

As an example it was found that in the lens molded body of Example 1 the ratio I_{Arm}/I_{CH} decreased rapidly in the order 1.50, 1.25, 1.16 and 1.09 from Region 1 to Region 4, as Table 1 shows. The line profile (b) in Fig. 4A revealed that the ratio I_{Arm}/I_{CH} took an a particularly high value in excess of 1.60 at a region about 5 µm from the surface, within Region A, and that the abundance ratio of EGPEMA fluctuated locally at the surface, as compared with the lens molded body of Example 2. Although I_{Arm}/I_{CH} changed rapidly at Regions 1 and 2, it was found that I_{Arm}/I_{CH} changed gently, with substantial convergence of fluctuations, at Region 3 and Region 4.

As an example, it was found that in the lens molded body of Example 2 the ratio I_{Arm}/I_{CH} increased sequentially from Region 1 to Region 4 in the order 0.54, 0.62, 0.69 and 0.72. It was found that the slope of the I_{Arm}/I_{CH} line was steep in Region 1 and Region 2, where the ratio of increase of the I_{Arm}/I_{CH} value was relatively high. In (b) of Fig. 4B, it is observed that I_{Arm}/I_{CH} increases little by little also in Region 3 and Region 4. However, a check of the Raman spectra themselves reveals that the Raman spectrum obtained for Region 3 (for instance point at 30 µm in the thickness direction) and the Raman spectrum obtained in Region 4 (for instance point at 40 µm in the thickness direction) did not exhibit significant differences. From the above it was found that although in the line profile in (b) of Fig. 4B the ratio I_{Arm}/I_{CH} is higher at Region 4 than in Region 3, this arises from the influence of the S/N ratio of the spectra, and actually the ratio I_{Arm}/I_{CH} does not exhibit a significant change in Region C and Region D, which are removed by about 20 µm or more from the surface.

### (Test example 2)

Herein there were measured surface tackiness and surface roughness of a lens molded body (Example) produced in the same way as in Example 1 and Example 2 of Test example 1. For the purpose of comparison, a soft acrylic base material was prepared through polymerization, into a plate shape, of a starting resin composition identical to that of Example 2, and the resulting base material was cut, to prepare a lens cutting (Comparative Example) of identical shape. The lens cutting exhibited, as it was, a very high tackiness, and accordingly was subjected to a plasma treatment in which the surface of the lens cutting was irradiated, after cutting, with plasma, to thereby reduce surface tackiness. The lens cutting (Comparative Example) having undergone the plasma treatment was retrieved, and tackiness and surface roughness were evaluated in the same way as in the above lens molded body.

### [Evaluation of tackiness of a lens molded body]

Tackiness was evaluated in accordance with the following method (so-called probe tack test method).

Specifically, a force gauge ZTA-20N by Imada Co., Ltd. was used as a tackiness measuring device, and a pin gauge (PG-5, rod outer diameter 5 mm, pressing area ∅4 mm) was used as a pressing indenter.

The force gauge was used by being set on a measuring stand (MX2-500N) in such a manner that the test direction matched a vertical downward direction. Then, in an environment of room temperature 23±0.5°C and relative humidity of about 40%, the lens molded body was disposed so that the center of a lens molded body was positioned directly below the pressing indenter, and the back surface of the lens molded body faced upward (measurement surface).

The pressing indenter was pressed against the lens molded body at an indentation rate of 15 mm/s, an indentation load of 5 N and for an indentation time of 10 seconds, after which there was measured peeling strength upon pull-up of the pressing indenter at a pull-up rate of 15 mm/s. The force gauge was driven using control software (Force Recorder Standard), peeling strength was measured with the number of data measurement points set to one data point every 0.01 seconds, and the arithmetic mean value and standard deviation of peel strength were calculated, with the number of tests set to N = 10; the results are given in Table 2.

### [Surface roughness of the lens molded body]

The three-dimensional shape of the surface of each of the lens molded bodies of Example 1 and Example 2 (Examples according to the present invention) and of Comparative Example were measured through scanning of a small 10 µm square region using a scanning probe microscope (SPM). From the obtained results there were calculated surface roughness parameters in the form of center line average roughness (average deviation, Ra), root mean square surface roughness (RMS) and surface area ratio (S Ratio); the results are given jointly in Table 2. These surface roughness parameters were measured in accordance with JIS B 0601 and JIS B 0633.

An intermolecular force probe microscope system (MFP-3D-SA) by Oxford Instruments PLC was used as the SPM in an air atmosphere, at room temperature (25°C), with the measurement mode set to DFM mode, and using Multi 75Al·G (spring constant 3 N/m) as a probe.

### [Table 2]

**Table 2**

| | Evaluation item | Example 1 | Example 2 | Comp. Example |
|---|---|---|---|---|
| Tackiness | Average peeling force (N) | 1.46 | 5.81 | 14.3 |
| | Adhesive strength(N/mm²) | 0.116 | 0.463 | 1.139 |
| | Standard deviation (-) | 0.07 | 0.30 | 2.52 |
| Surface roughness | Ra [nm] | 0.180 | 0.194 | 0.372 |
| | RMS [nm] | 0.228 | 0.244 | 0.470 |
| | S Ratio [-] | 1.00072 | 1.00241 | 1.00437 |

As Table 2 shows, the lens molded bodies of Examples 1 and 2 exhibited stable and sufficiently reduced surface tackiness as compared with a cut product having undergone plasma irradiation in Comparative Example. For instance, adhesive strength was specifically about 1.14 N/mm² also for a cut product having undergone plasma irradiation for reducing tackiness, but by contrast was about 0.12 N/mm² for the lens molded body of Example 1, and about 0.46 N/mm² for the lens molded body of Example 2. It was found that the tackiness of the lens molded body of Example 2 and the lens cut body of Comparative Example could be reduced to about 40% (reduction rate of about 60%) by using a same resin starting material composition while controlling the manner in which the resin starting material composition is polymerized. The cut product of Comparative Example was roughened to a surface roughness of 0.372 nm, as a result of plasma irradiation for reducing tackiness. In the lens molded body of Example 2, by contrast, it was found that surface tackiness was reduced without performing a particular surface treatment; accordingly surface roughness could be reduced to a very low value, of 0.194 nm, corresponding to the casting mold. It was found that the surface roughness of the lens molded body of Example 1 in which the monomer species have been adjusted, was comparable to that of the lens molded body of Example 2, but tackiness was further reduced than in the lens molded body of Example 2. It was seen that the surface tackiness of the lens molded body according to the art disclosed herein can be kept sufficiently low, without any particular processing, while the lens molded body exhibits sufficient flexibility and shape recovery. Accordingly, it was found that the lens molded body has a smooth surface, of small surface roughness, for instance 0.3 nm or less (for instance 0.25 nm or less).

### (Test example 2)

### [Preparation of an intraocular lens]

A starting material composition for intraocular lens was prepared in the same way as in Example 2 of Test example 1. The starting material composition was injected into a casting mold provided with a pier, for a biconvex lens shape having a diameter of 6 mm and a thickness of 0.56 mm, and thermal polymerization was carried out within an air oven, at conditions of 60°C for 12 hours, and 100°C for 12 hours, to thereby obtain lens molded bodies of Examples 2-1 to 2-3. Three casting molds were prepared out of glass, SUS and PEEK, and the upper and lower dies were combined in the manner given in Table 3.

### [Measurement of refractive index]

The refractive index on the front surface and the back surface of the prepared lens molded bodies of Examples 2-1 to 2-3 was measured. The results are given jointly in Table 3. The positions for measuring refractive index were set, in a plan view, at a center point of the circular lens optical portion, on the top surface in contact with the upper die at the time of polymerization, and the bottom surface in contact with the bottom die. The refractive index at room temperature (23°C) was measured using a digital refractometer (RX-7000i, by Atago Co., Ltd.).

### [Table 3]

**Table 3**

| No. | Casting mold material/refractive index | | | |
|---|---|---|---|---|
| | Top surface | Refractive index | Bottom surface | Refractive index |
| 2-1 | Glass | 1.51778 | Glass | 1.51783 |
| 2-2 | SUS | 1.51824 | SUS | 1.51834 |
| 2-3 | PEEK | 1.50798 | Glass | 1.51843 |

As Table 3 shows, it was found that the refractive index of the lens molded body lay in the range of 1.517 to 1.519, at the top surface and bottom surface of Examples 2-1 to 2-2, in which glass and SUS were used as a casting mold, and at the bottom surface of Example 2-3. In particular, the difference in refractive index between the top surface and bottom surface was small, namely 0.00005 and 0.00010 in Example 2-1 and Example 2-2, respectively. Although not specifically shown, it was observed that the refractive index at the thickness-direction center of a lens molded body obtained through curing of a starting material composition identical to that of the Examples lay for instance within a range of about 1.5185±0.0020.

It was found that, by contrast, the surface refractive index of the lens molded body dropped significantly below 1.517, for instance to about 1.508±0.002, at the top surface of Example 2-3 in which a casting mold containing an organic compound such as PEEK was used. The difference in refractive index between the top surface and the bottom surface in Example 2-3 was 0.01045, markedly larger than that in Example 2-1 and Example 2-2. Although not specifically shown, the refractive index at the thickness-direction center of the lens molded body of Example 2-3 lay throughout within the range 1.5185±0.0020, similarly to those of Example 2-1 Example 2-2.

Herein EGPEA (liquid) used as one main monomer, from among the starting material monomers, has an aromatic ring within the molecular structure, and hence the refractive index is relatively high, of 1.5180, whereas the refractive index of the alkyl methacrylate (liquid) likewise used as main monomer is relatively low, of 1.4230. It is known that the oxygen permeability of an organic compound such as PEEK is higher than that of inorganic materials such as glass and SUS. From the above it can be concluded that through the use of a casting mold made of an organic compound such as PEEK having high oxygen permeability, polymerization of the alkyl methacrylate progresses dominantly, among the acrylate and methacrylate-based copolymers that are polymerized at the surface in contact with the casting mold, and as a result an alkyl methacrylate component is predominantly present at the surface portion in contact with the casting mold. In other words, it is found that a methacrylate-rich layer is formed at the surface portion in contact with the casting mold. It is deemed that this is accompanied by a relatively lower refractive index at the surface portion of the lens molded body than at the central portion, on account of the high content ratio of the alkyl methacrylate component. Although not specifically shown, a large change in refractive index such as that of the lens molded body of Example 2, could not be observed between the surface portion and the central portion of the lens molded body of Example 1, which exhibited a refractive index of about 1.52 to 1.53. As a conceivable underlying reason, it is conjectured that possibly there arose no significant refractive index difference with respect to the acrylic-based monomer component due to the fact that the methacrylate-based monomer component of relatively low refractive index has an aromatic ring of relatively high refractive index.

Concrete examples of the present invention have been explained in detail above, but these are merely illustrative in nature, and are not meant to limit the claims in any way. The art set forth in the claims accommodates various modifications and alterations of the concrete examples illustrated above.

### Reference Signs List

- 1: Intraocular lens
- 10: Optical portion
- 12: Surface portion
- 14: Central portion
- 20: Support portion

## Claims

1. An intraocular lens composed of a copolymer that includes an acrylate-based monomer component A and a methacrylate-based monomer component B as constituent components, the intraocular lens comprising:
a surface portion that includes a surface of the copolymer; and
a central portion closer to a center than the surface portion,
wherein the surface portion forms a methacrylate-rich layer containing a higher content of the monomer component B, as a monomer component, than that in the central portion.

2. The intraocular lens of claim 1,
wherein the acrylate-based monomer component A includes an alkoxy group-containing acrylate,
the methacrylate-based monomer component B includes an aromatic ring-containing methacrylate, and
the intraocular lens further comprises a hydrophilic hydroxy group-containing acrylate as a monomer component.

3. The intraocular lens of claim 2,
wherein the alkoxy group-containing acrylate includes an acrylate represented by General formula (1) below: 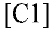 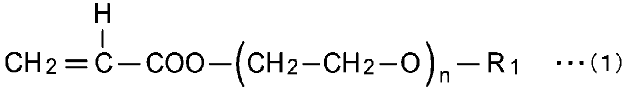
wherein, R1 is a methyl group or ethyl group, and n is an integer from 1 to 4,
the aromatic ring-containing methacrylate includes a methacrylate represented by General formula (2) below: 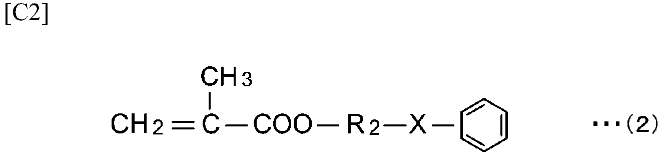
wherein, R2 is a C₁₋₈ linear or branched alkylene group, and X is absent or represents an oxygen atom, and
in a Raman spectroscopic analysis spectrum at an excitation wavelength of 532 nm,
when a ratio of an intensity I_{Arm}, of a Raman peak derived from an aromatic ring observed in a vicinity of 1003 cm⁻¹ with respect to an intensity I_{CH} of a Raman peak derived from a C-H bond observed in a vicinity of 1450 cm⁻¹ is represented by R = I_{Arm}/I_{CH},
a ratio Rs₁ at the surface portion of the intraocular lens and a ratio Rc₁ at the central portion of the intraocular lens satisfy a relationship of Rs₁ ≥ 1.1 × Rc₁.

4. The intraocular lens of claim 2or 3,
wherein among the monomer components that constitute the copolymer,
the aromatic ring-containing methacrylate is contained in an amount of at least 40 mass% and at most 52 mass%,
the alkoxy group-containing acrylate is contained in an amount of at least 35 mass% and at most 46 mass%, and
the hydroxy group-containing acrylate is contained in an amount of at least 8 mass% and at most 12 mass%.

5. The intraocular lens of claim 1,
wherein the acrylate-based monomer component A includes a compound represented by General formula (3) below: 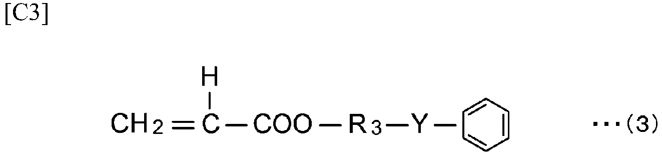
wherein, R3 is a C₁₋₆ linear or branched alkylene group, and Y is absent or represents an oxygen atom,
the methacrylate-based monomer component B includes a compound represented by General formula (4) below: 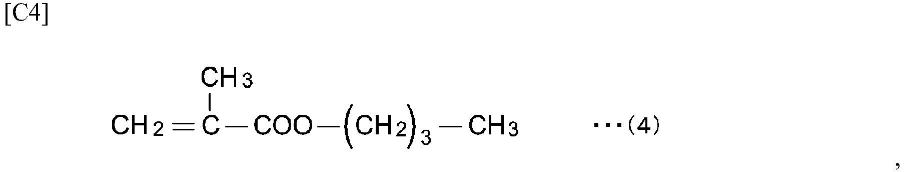 and
in a Raman spectroscopic analysis spectrum at an excitation wavelength of 532 nm,
when a ratio of an intensity I_{Arm}, of a Raman peak derived from an aromatic ring observed in a vicinity of 1000 cm⁻¹ with respect to an intensity I_{CH} of a Raman peak derived from a C-H bond observed in a vicinity of 1452 cm⁻¹ is presented by R = I_{Arm}/I_{CH},
a ratio Rs₂ at the surface portion of the intraocular lens and a ratio Rc₂ at the central portion of the intraocular lens satisfy a relationship of Rs₂ ≤ 0.95 × Rc₂.

6. The intraocular lens of claim 5,
wherein among the monomer components that constitute the copolymer,
a proportion of the acrylate-based monomer component A in all monomer components is at least 42 mass% and at most 62 mass%, and
a proportion of the methacrylate-based monomer component B in all monomer components is at least 35 mass% and at most 55 mass%.

7. The intraocular lens of claim 5 or 6,
wherein the surface portion has a refractive index of at least 1.505 and less than 1.51, and
the central portion has a refractive index of at least 1.51 and at most 1.53.

8. The intraocular lens of claim 3 or 5, wherein the ratio R changes continuously from the central portion towards the surface of the intraocular lens.

9. The intraocular lens of any one of claims 1 to 8, further comprising a monomer component C as a monomer component that constitutes the copolymer, the monomer component C including a functional group having ultraviolet absorbing ability.

10. The intraocular lens of claim 9, wherein the monomer component C is contained in a proportion of at least 0.1 mass% and at most 1.5 mass% relative to a total amount of the acrylate-based monomer component A and the methacrylate-based monomer component B.

11. The intraocular lens of any one of claims 1 to 10, wherein the surface portion has a thickness of at least 1 µm and at most 40 µm, the thickness being a dimension in a direction perpendicular to the surface.

12. The intraocular lens of any one of claims 1 to 11, comprising
a circular optical portion, and a pair of support portions that support the optical portion,
wherein the optical portion and the support portions are formed integrally.

13. An intraocular lens insertion system, comprising:
the intraocular lens of any one of claims 1 to 12; and
an injector,
wherein the intraocular lens is accommodated beforehand in the injector.

## Patentansprüche

1. Intraokularlinse, bestehend aus einem Copolymer, das einen acrylatbasierten Monomerbestandteil A und einen methacrylatbasierten Monomerbestandteil B als Bestandteile aufweist, wobei die Intraokularlinse aufweist:
einen Oberflächenabschnitt, der eine Oberfläche des Copolymers beinhaltet; und einen Zentralabschnitt, der näher an einem Zentrum liegt als der Oberflächenabschnitt,
wobei der Oberflächenabschnitt eine methacrylatreiche Schicht bildet, die einen höheren Gehalt des Monomerbestandteils B als einen Monomerbestandteil enthält als derjenige in dem Zentralabschnitt.

2. Intraokularlinse nach Anspruch 1,
wobei der acrylatbasierte Monomerbestandteil A ein alkoxygruppenhaltiges Acrylat beinhaltet,
wobei der methacrylatbasierte Monomerbestandteil B ein Methacrylat, das einen aromatischen Ring enthält, beinhaltet, und
wobei die Intraokularlinse ferner ein hydrophiles hydroxygruppenhaltiges Acrylat als einen Monomerbestandteil aufweist.

3. Intraokularlinse nach Anspruch 2,
wobei das alkoxygruppenhaltige Acrylat ein Acrylat beinhaltet, das durch die untenstehende allgemeine Formel (1) dargestellt wird: 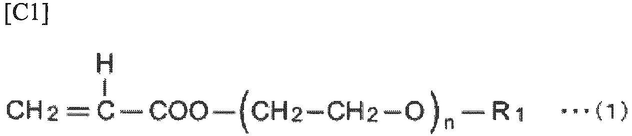
wobei R1 eine Methylgruppe oder eine Ethylgruppe ist, und n eine Ganzzahl von 1 bis 4 ist, wobei das Methacrylat, das einen aromatischen Ring enthält, ein Methacrylat beinhaltet, das durch die untenstehende allgemeine Formal (2) dargestellt wird: 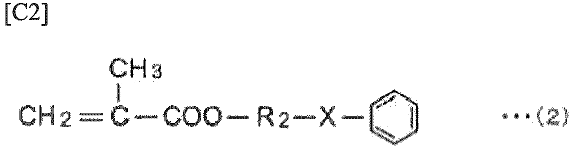
wobei R2 eine lineare oder verzweigte C₁₋₈-Alkylengruppe ist und X abwesend ist oder ein Sauerstoffatom darstellt, und
in einem Raman-Spektroskopie-Analysespektrum bei einer Anregungswellenlänge von 532 nm,
wenn ein Verhältnis einer Intensität I_{Arm} eines Ramanpeaks, der von einem aromatischen Ring abgeleitet wird, der in einer Nähe von 1003 cm⁻¹ beobachtet wird, im Verhältnis zu einer Intensität I_{CH} eines Ramanpeaks, der von einer C-H-Bindung abgeleitet wird, der in einer Nähe von 1450 cm⁻¹ beobachtet wird, durch R=I_{Arm}/I_{CH} dargestellt wird,
ein Verhältnis Rsi an dem Oberflächenabschnitt der Intraokularlinse und ein Verhältnis Rc₁ an dem Zentralabschnitt der Intraokularlinse ein Verhältnis von Rs₁≥1,1×Rc₁ erfüllen.

4. Intraokularlinse nach Anspruch 2 oder 3,
wobei von den Monomerbestandteilen, aus denen das Copolymer besteht, das Methacrylat, das einen aromatischen Ring enthält, in einer Menge von zumindest 40 Masseprozent und höchstens 52 Masseprozent enthalten ist,
das alkoxygruppenhaltige Acrylat in einer Menge von zumindest 35 Masseprozent und höchstens 46 Masseprozent enthalten ist, und
das hydroxygruppenhaltige Acrylat in einer Menge von zumindest 8 Masseprozent und höchstens 12 Masseprozent enthalten ist.

5. Intraokularlinse nach Anspruch 1,
wobei der acrylatbasierte Monomerbestandteil A eine Verbindung beinhaltet, die durch die untenstehende allgemeine Formel (3) dargestellt wird: 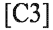 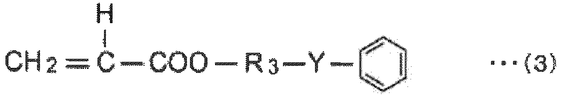
wobei R3 eine lineare oder verzweigte C₁₋₆-Alkylengruppe ist und Y abwesend ist oder ein Sauerstoffatom darstellt,
der methacrylatbasierte Monomerbestandteil B eine Verbindung beinhaltet, die durch die untenstehende allgemeine Formal (4) dargestellt wird: 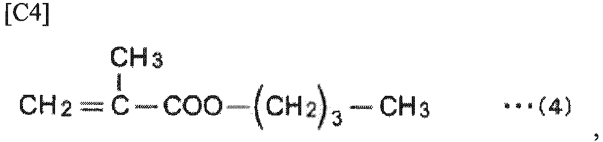 und
in einem Raman-Spektroskopie-Analysespektrum bei einer Anregungswellenlänge von 532 nm,
wenn ein Verhältnis einer Intensität I_{Arm} eines Ramanpeaks, der von einem aromatischen Ring abgeleitet wird, der in einer Nähe von 1000 cm⁻¹ beobachtet wird, im Verhältnis zu einer Intensität I_{CH} eines Ramanpeaks, der von einer C-H-Bindung abgeleitet wird, der in einer Nähe von 1452 cm⁻¹ beobachtet wird, durch R=I_{Arm}/I_{CH} dargestellt wird,
ein Verhältnis Rs₂ an dem Oberflächenabschnitt der Intraokularlinse und ein Verhältnis Rc₂ an dem Zentralabschnitt der Intraokularlinse ein Verhältnis von Rs₂≤0,95×Rc₂ erfüllen.

6. Intraokularlinse nach Anspruch 5,
wobei von den Monomerbestandteilen, aus denen das Copolymer besteht, ein Anteil des acrylatbasierten Monomerbestandteils A in allen Monomerbestandteilen zumindest 42 Masseprozent und höchstens 62 Masseprozent beträgt, und
ein Anteil des methacrylatbasierten Monomerbestandteils B in allen Monomerbestandteilen zumindest 35 Masseprozent und höchstens 55 Masseprozent beträgt.

7. Intraokularlinse nach Anspruch 5 oder 6,
wobei der Oberflächenabschnitt einen Brechungsindex von zumindest 1,505 und weniger als 1,51 aufweist, und
der Zentralabschnitt einen Brechungsindex von zumindest 1,51 und höchstens 1,53 aufweist.

8. Intraokularlinse nach Anspruch 3 oder 5, wobei das Verhältnis R sich kontinuierlich von dem Zentralabschnitt in Richtung der Oberfläche der Intraokularlinse ändert.

9. Intraokularlinse nach einem der Ansprüche 1 bis 8, ferner aufweisend einen Monomerbestandteil C als einen Monomerbestandteil, aus dem das Copolymer besteht, wobei der Monomerbestandteil C eine funktionale Gruppe beinhaltet, die eine UV-Absorptionsfähigkeit besitzt.

10. Intraokularlinse nach Anspruch 9, wobei der Monomerbestandteil C in einem Anteil von mindestens 0,1 Masseprozent und höchstens 1,5 Masseprozent im Verhältnis zu einer Gesamtmenge des acrylatbasierten Monomerbestandteils A und des methacrylatbasierten Monomerbestandteils B enthalten ist.

11. Intraokularlinse nach einem der Ansprüche 1 bis 10, wobei der Oberflächenabschnitt eine Dicke von mindestens 1 µm und höchstens 40 µm aufweist, wobei die Dicke eine Abmessung in einer Richtung ist, die zu der Oberfläche rechtwinklig ist.

12. Intraokularlinse nach einem der Ansprüche 1 bis 11, aufweisend:
einen kreisförmigen Optikabschnitt und ein Paar Trageabschnitte, die den Optikabschnitt tragen,
wobei der Optikabschnitt und die Trageabschnitte einstückig ausgebildet sind.

13. Intraokularlinseneinfügesystem, aufweisend:
die Intraokularlinse nach einem der Ansprüche 1 bis 12; und
einen Injektor,
wobei die Intraokularlinse im Vorfeld in dem Injektor akkommodiert wird.

## Revendications

1. Lentille intraoculaire constituée d'un copolymère qui inclut un constituant monomère à base d'acrylate A et un constituant monomère à base de méthacrylate B comme constituants de constitution, la lentille intraoculaire comprenant :
une portion de surface qui inclut une surface du copolymère ; et
une portion centrale plus proche d'un centre que la portion de surface,
dans laquelle la portion de surface forme une couche riche en méthacrylate contenant une teneur plus élevée du constituant monomère B, comme un constituant monomère, que celle dans la portion centrale.

2. Lentille intraoculaire selon la revendication 1,
dans laquelle le constituant monomère à base d'acrylate A inclut un acrylate contenant un groupe alcoxy,
le constituant monomère à base de méthacrylate B inclut un méthacrylate contenant un noyau aromatique, et
la lentille intraoculaire comprend de plus un acrylate contenant un groupe hydroxy hydrophile comme un constituant monomère.

3. Lentille intraoculaire selon la revendication 2,
dans laquelle l'acrylate contenant un groupe alcoxy inclut un acrylate représenté par la formule générale (1) ci-dessous : 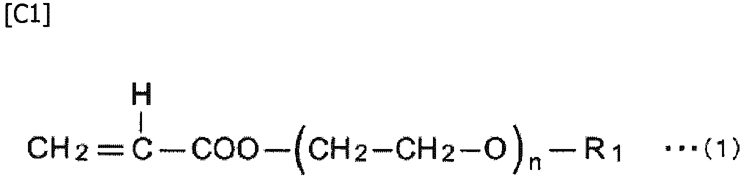
dans laquelle, R¹ est un groupe méthyle ou groupe éthyle, et n est un nombre entier de 1 à 4,
le méthacrylate contenant un noyau aromatique inclut un méthacrylate représenté par la formule générale (2) ci-dessous : 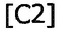 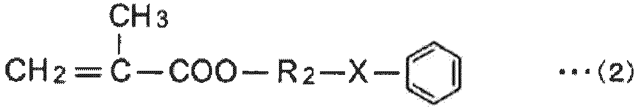
dans laquelle, R2 est un groupe alkylène linéaire ou ramifié en C₁₋₈, et X est absent ou représente un atome d'oxygène, et
dans un spectre d'analyse spectroscopique Raman à une longueur d'onde d'excitation de 532 nm,
lorsqu'un rapport d'une intensité I_{Arm} d'un pic Raman dérivé d'un noyau aromatique observé au voisinage de 1 003 cm⁻¹ par rapport à une intensité I_{CH} d'un pic Raman dérivé d'une liaison C-H observé au voisinage de 1 450 cm⁻¹ est représenté par R = I_{Arm}/I_{CH},
un rapport Rs₁ sur la portion de surface de la lentille intraoculaire et un rapport Rc₁ sur la portion centrale de la lentille intraoculaire satisfont une relation de Rs₁ ≥ 1,1 × Rc₁.

4. Lentille intraoculaire selon la revendication 2 ou 3,
dans laquelle parmi les constituants monomères qui constituent le copolymère,
le méthacrylate contenant un noyau aromatique est contenu dans une quantité d'au moins 40 % en masse et d'au plus 52 % en masse,
l'acrylate contenant un groupe alcoxy est contenu dans une quantité d'au moins 35 % en masse et d'au plus 46 % en masse, et
l'acrylate contenant un groupe hydroxy est contenu dans une quantité d'au moins 8 % en masse et d'au plus 12 % en masse.

5. Lentille intraoculaire selon la revendication 1,
dans laquelle le constituant monomère à base d'acrylate A inclut un composé représenté par la formule générale (3) ci-dessous : 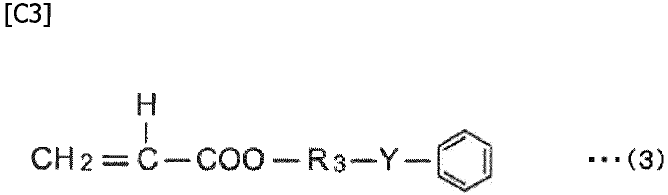
dans laquelle, R3 est un groupe alkylène linéaire ou ramifié en C₁₋₆, et Y est absent ou représente un atome d'oxygène,
le constituant monomère à base de méthacrylate B inclut un composé représenté par la formule générale (4) ci-dessous : 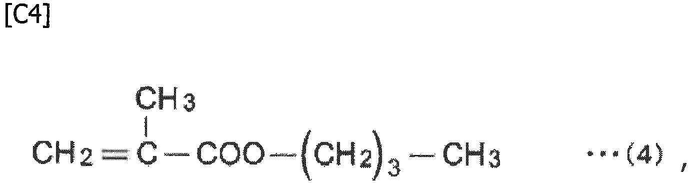 et
dans un spectre d'analyse spectroscopique Raman à une longueur d'onde d'excitation de 532 nm,
lorsqu'un rapport d'une intensité I_{Arm} d'un pic Raman dérivé d'un noyau aromatique observé au voisinage de 1 000 cm⁻¹ par rapport à une intensité I_{CH} d'un pic Raman dérivé d'une liaison C-H observé au voisinage de 1 452 cm⁻¹ est représenté par R = I_{Arm/}I_{CH},
un rapport Rs₂ sur la portion de surface de la lentille intraoculaire et un rapport Rc₂ sur la portion centrale de la lentille intraoculaire satisfont une relation de Rs₂ ≤ 0,95 × Rc₂.

6. Lentille intraoculaire selon la revendication 5,
dans laquelle parmi les constituants monomères qui constituent le copolymère,
une proportion du constituant monomère à base d'acrylate A dans tous les constituants monomères est d'au moins 42 % en masse et d'au plus 62 % en masse, et
une proportion du constituant monomère à base de méthacrylate B dans tous les constituants monomères est d'au moins 35 % en masse et d'au plus 55 % en masse.

7. Lentille intraoculaire selon la revendication 5 ou 6,
dans laquelle la portion de surface présente un indice de réfraction d'au moins 1,505 et inférieur à 1,51, et
la portion centrale présente un indice de réfraction d'au moins 1,51 et d'au plus 1,53.

8. Lentille intraoculaire selon la revendication 3 ou 5, dans laquelle le rapport R change en continu à partir de la portion centrale vers la surface de la lentille intraoculaire.

9. Lentille intraoculaire selon l'une quelconque des revendications 1 à 8, comprenant de plus un constituant monomère C comme un constituant monomère qui constitue le copolymère, le constituant monomère C incluant un groupe fonctionnel ayant une aptitude d'absorption des ultraviolets.

10. Lentille intraoculaire selon la revendication 9, dans laquelle le constituant monomère C est contenu dans une proportion d'au moins 0,1 % en masse et d'au plus 1,5 % en masse par rapport à une quantité totale du constituant monomère à base d'acrylate A et du constituant monomère à base de méthacrylate B.

11. Lentille intraoculaire selon l'une quelconque des revendications 1 à 10, dans laquelle la portion de surface présente une épaisseur d'au moins 1 µm et d'au plus 40 µm, l'épaisseur étant une dimension dans une direction perpendiculaire à la surface.

12. Lentille intraoculaire selon l'une quelconque des revendications 1 à 11, comprenant
une portion optique circulaire, et une paire de portions de support qui supportent la portion optique,
dans laquelle la portion optique et les portions de support sont formées d'une seule pièce.

13. Système d'insertion de lentille intraoculaire, comprenant :
la lentille intraoculaire selon l'une quelconque des revendications 1 à 12 ; et
un injecteur,
dans lequel la lentille intraoculaire est logée auparavant dans l'injecteur.
